(19) Europäisches Patentamt
European Patent Office
Office européen des brevets

(11) **EP 4 728 989 A1**

(12) **EUROPEAN PATENT APPLICATION**
published in accordance with Art. 153(4) EPC

(43) Date of publication:
**22.04.2026 Bulletin 2026/17**

(21) Application number: **24823604.4**

(22) Date of filing: **27.05.2024**

(51) International Patent Classification (IPC):
**A61B 6/58** (2024.01)   **A61B 6/00** (2024.01)

(52) Cooperative Patent Classification (CPC):
**A61B 6/589; A61B 6/00; A61B 6/08; A61B 6/466;**
**A61B 6/542; A61B 6/544; A61B 6/58; A61B 6/587;**
**A61B 6/588**

(86) International application number:
**PCT/KR2024/007155**

(87) International publication number:
**WO 2024/258083 (19.12.2024 Gazette 2024/51)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **15.06.2023 KR 20230076519**

(71) Applicant: **DRTECH CORPORATION**
**Seongnam-si, Gyeonggi-do 13216 (KR)**

(72) Inventors:
• **LIM, Sung Hoon**
  **Yongin-si Gyeonggi-do 16908 (KR)**
• **KIM, Tae Hyung**
  **Seongnam-si Gyeonggi-do 13165 (KR)**
• **SHIN, Choul Woo**
  **Seongnam-si Gyeonggi-do 13476 (KR)**

(74) Representative: **Jung, Minkyu**
**Wuesthoff & Wuesthoff**
**Patentanwälte und Rechtsanwalt PartG mbB**
**Schweigerstraße 2**
**81541 München (DE)**

(54) **MOBILE MEDICAL IMAGING DEVICE AND OPERATING METHOD OF MEDICAL IMAGING DEVICE**

(57)     A medical imaging device of the present disclosure includes a detector including a detector controller for controlling an operation of the detector and a first transceiver disposed at a predetermined position, a source assembly including a second transceiver disposed on a front surface and a 3D camera, and a main controller controlling an operation of the medical imaging device. When an intensity of a signal from the first transceiver that is received by the second transceiver is higher than a predetermined threshold signal intensity, the main controller activates the medical imaging device, obtains a 3D camera image of at least one of a subject and the detector based on the 3D camera, displays a radiation irradiation region in the 3D camera image based on state information of the source assembly, displays a region of the detector in the 3D camera image, outputs a message that guides to adjust the irradiation region and the region of the detector, and when the region of the detector and the irradiation region coincide, transmits a radiation irradiation ready signal to the first transceiver using the second transceiver.

Fig. 1

**Description**

[Technical Field]

The present disclosure relates to a medical imaging device and an operating method of the medical imaging device. More specifically, the present disclosure includes a method in which a source assembly of a medical imaging device is aligned with a detector, thereby obtaining a clear radiation image.

[Background Art]

Aligning a medical imaging device and a detector is an important step for obtaining a high-quality image and minimizing exposure to radiation for both a patient and a medical service provider. The following steps may be performed to align a medical imaging device and a detector.

First, the position of a source assembly may be determined. The source assembly for generating radiation should be located at a fixed distance from a region of interest of a patient. The distance varies according to the type of medical imaging device and a region being imaged but is usually about 1 to 2 meters. Next, a radiation beam may be aligned. A radiation beam should be aligned to be perpendicular to a detector and pass through the region of interest of the patient. The alignment may be performed by adjusting the position of the source assembly or forming the radiation beam using a collimator.

Next, the position of the detector may be designated. The detector should be located at the opposite side of the patient relative to the source assembly and should be aligned with the radiation beam. In addition, the detector may be disposed as close as possible to the patient to minimize scattered radiation and improve image quality. Lastly, an alignment checking step may be performed. When the source assembly and the detector are at their correct positions, a test image may be captured, and the alignment may be checked. Whether the region of interest is at the center of the image and the image quality is sufficient for diagnosis may be checked.

To sum up, appropriate alignment between a mobile medical imaging device and a detector is important in obtaining an accurate, high-quality image. Therefore, development of various devices for precisely guiding the alignment is underway.

[Disclosure]

[Technical Problem]

The present disclosure discloses a medical imaging device that allows precise alignment between a source assembly of a mobile medical imaging device and a detector. However, objectives are not limited to that mentioned above, and other objectives may be present.

[Technical Solution]

A medical imaging device according to the present disclosure includes a detector including a detector controller for controlling an operation of the detector and a first transceiver disposed at a predetermined position, a source assembly including a second transceiver disposed on a front surface, and a main controller controlling an operation of the medical imaging device, wherein the main controller obtains alignment information for alignment between the detector and the source assembly.

The alignment information of the medical imaging device according to the present disclosure may include information related to at least one of a direction, a distance, and an angle of movement of the source assembly that is necessary, based on at least one of posture information of the detector and posture information of the source assembly.

The medical imaging device according to the present disclosure may, when a region of the detector and an irradiation region coincide based on the alignment information, output a signal indicating that the region of the detector and the irradiation region coincide.

The detector of the medical imaging device according to the present disclosure may include a plurality of first transceivers, the source assembly may include a plurality of second transceivers, and the main controller may obtain a source-to-image distance (SID) from the source assembly to the detector based on a plurality of distances between the plurality of first transceivers and the plurality of second transceivers that correspond one-to-one.

The source assembly of the medical imaging device according to the present disclosure may further include a 3D camera for obtaining a 3D camera image, and the main controller may obtain at least one of the posture information of the source assembly and the posture information of the detector based on the 3D camera image.

The main controller of the medical imaging device according to the present disclosure may obtain pre-alignment information using the first transceiver and the second transceiver, may obtain post-alignment information based on the 3D camera image, and may determine final alignment information based on the pre-alignment information and the post-alignment information.

The medical imaging device according to the present disclosure may obtain first distance information between the first transceiver and the second transceiver based on a signal between the first transceiver and the second transceiver and may display the region of the detector on the 3D camera image based on the first distance information.

The main controller of the medical imaging device according to the present disclosure may display the region of the detector in the 3D camera image, may obtain second distance information from the 3D camera to at least one image-captured object, and may obtain a source-to-image distance (SID) from the source assembly to the detector based on the region of the detector and the second distance information.

The main controller of the medical imaging device according to the present disclosure may obtain second distance information from the 3D camera to at least one image-captured object and may obtain a source-to-object distance (SOD) from the source assembly to a subject based on the second distance information.

The main controller of the medical imaging device according to the present disclosure may obtain first distance information between the first transceiver and the second transceiver based on a signal between the first transceiver and the second transceiver, may obtain a source-to-image distance (SID) from the source assembly to the detector based on the first distance information, and may determine a thickness of the subject based on the SOD and the SID.

The main controller of the medical imaging device according to the present disclosure may obtain body part identification information of the subject, may obtain a body part model by modeling the subject three-dimensionally or two-dimensionally based on the body part identification information and the SOD, may place the center of the region of the detector at the center of the body part model shown in the 3D camera image, and may display the region of the detector based on the SID.

The 3D camera of the medical imaging device according to the present disclosure may include at least one of a depth measurement camera, a red-green-blue-depth (RGBD) camera, or a time-of-flight (TOF) camera.

In addition, a program for implementing an operation method of the medical imaging device described above may be recorded in a computer-readable recording medium.

[Advantageous Effects]

By providing a means for promptly aligning a source assembly and a detector of a medical imaging device of the present disclosure, user convenience can be enhanced, and medical image quality can be improved.

Advantageous effects that can be obtained by the present disclosure are not limited to that mentioned above, and other unmentioned advantageous effects should be clearly understood by those of ordinary skill in the art to which the present disclosure pertains from the following description.

[Description of Drawings]

FIG. 1 is a view showing a mobile medical imaging device according to one embodiment of the present disclosure.
FIG. 2 is a view showing a process of using the medical imaging device according to one embodiment of the present disclosure.
FIG. 3 is a view showing a block diagram of various components that may be included in the medical imaging device according to one embodiment of the present disclosure.
FIG. 4 shows a detector according to one embodiment of the present disclosure.
FIG. 5 is a view for describing a source assembly according to one embodiment of the present disclosure.
FIG. 6 is a flowchart for describing an operation of the medical imaging device according to one embodiment of the present disclosure.
FIG. 7 is a view for describing a medical imaging device according to one embodiment of the present disclosure.
FIG. 8 is a flowchart for describing a method of obtaining a region of the detector from a 3D camera image according to one embodiment of the present disclosure.
FIG. 9 is a view for describing an operation of the medical imaging device according to one embodiment of the present disclosure.
FIG. 10 is a view for describing an operation of the medical imaging device according to one embodiment of the present disclosure.
FIG. 11 is a view for describing an operation of the medical imaging device according to one embodiment of the present disclosure.
FIG. 12 is a view for describing an operation of the medical imaging device according to one embodiment of the present disclosure.

FIG. 13 is a view for describing an operation of the medical imaging device according to one embodiment of the present disclosure.

FIG. 14 is a view for describing an operation of the medical imaging device according to one embodiment of the present disclosure.

[Modes of the Invention]

Advantages and features of the disclosed embodiments and methods for achieving them will become clear by referring to the embodiments described below in conjunction with the accompanying drawings. However, the present disclosure is not limited to the embodiments disclosed below and may be implemented in various different forms, and these embodiments are provided only to make the disclosure complete and fully inform those of ordinary skill in the art to which the present disclosure pertains of the scope of the invention.

Terms used in this specification will be briefly described, and the disclosed embodiments will be described in detail.

General terms that are currently widely used have been selected as terms used in this specification in consideration of functions in the present disclosure, but the terms may vary depending on the intentions of engineers working in the related fields, precedents, the emergence of new technologies, or the like. Additionally, there may be terms deliberately selected by the applicants, and in such a case, their meanings will be described in detail in the corresponding part of the description of the invention. Accordingly, the terms used in this disclosure should be defined based on the meanings of the terms and the overall content of the present disclosure, rather than simply the names of the terms.

In this specification, a singular expression includes a plural expression unless the context clearly indicates singularity. Also, a plural expression includes a singular expression unless the context clearly indicates plurality.

Throughout the specification, when a certain part is described as "including" a certain component, this indicates that the certain part may further include other components instead of excluding other components unless the context clearly indicates otherwise.

Also, the term "-er/-or" or "part" used in this specification refers to a software or hardware component, and an "-er/-or" or "part" performs certain roles. However, the meaning of "-er/-or" or "part" is not limited to software or hardware. An "-er/-or" or "part" may be configured to be present in addressable storage media or configured to replay one or more processors. Therefore, as one example, an "-er/-or" or "part" may include components such as software components, object-oriented software components, class components, and task components, processes, functions, attributes, procedures, subroutines, program code segments, drivers, firmware, micro codes, circuits, data, databases, data structures, tables, arrays, or variables. The components and functions provided in "-ers/-ors" or "parts" may be combined into smaller numbers of components and "-ers/-ors" or "parts" or may be further separated into larger numbers of components and "-ers/-ors" or "parts."

According to one embodiment of the present disclosure, an "-er/-or" or "part" may be implemented using a processor and a memory. The term "processor" should be interpreted in a wide sense to include a universal processor, a central processing unit (CPU), a microprocessor, a digital signal processor (DSP), a controller, a microcontroller, a state machine, and the like. In some environments, "processor" may also refer to an application-specific integrated circuit (ASIC), a programmable logic device (PLD), a field programmable gate array (FPGA), or the like. For example, the term "processor" may also refer to a combination of processing devices such as a combination of a DSP and a microprocessor, a combination of a plurality of microprocessors, a combination of one or more microprocessors combined with a DSP core, or a combination of other arbitrary configurations.

The term "memory" should be interpreted in a wide sense to include an arbitrary electronic component that can store electronic information. The term "memory" may also refer to various types of processor-readable media such as a random access memory (RAM), a read-only memory (ROM), a nonvolatile random access memory (NVRAM), a programmable read-only memory (PROM), an erasable-programmable read-only memory (EPROM), an electrically erasable PROM (EEPROM), a flash memory, a magnetic or optical data storage device, and registers. When a processor is able to read information from a memory and/or record information in the memory, the memory is referred to as being in an electronic communication state with the processor. A memory integrated in a processor is in an electronic communication state with the processor.

Hereinafter, embodiments will be described in detail with reference to the accompanying drawings to allow those of ordinary skill in the art to which the present disclosure pertains to easily carry out the embodiments. Also, parts unrelated to the description are omitted from the drawings to clearly describe the present disclosure.

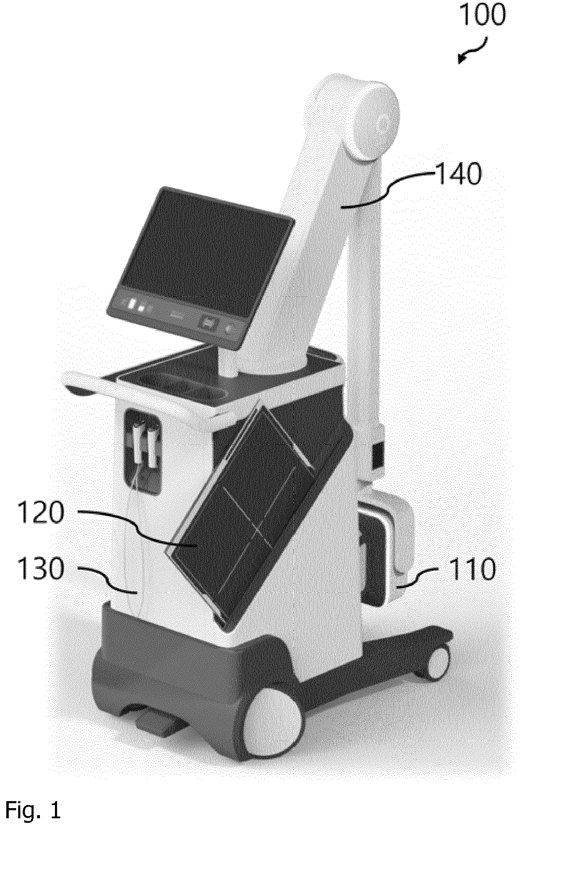

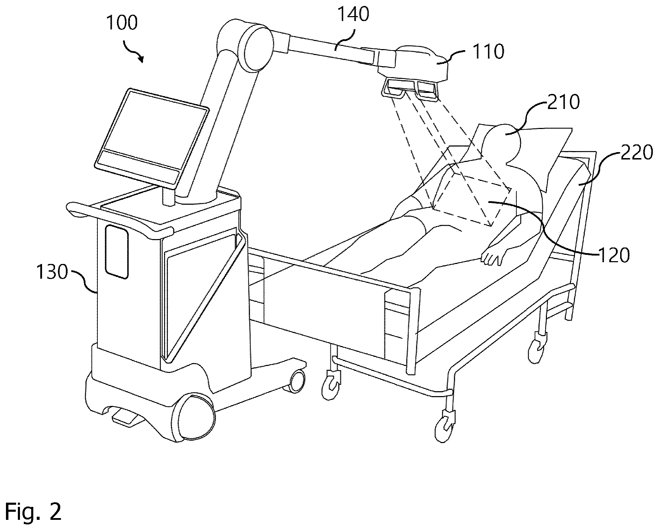

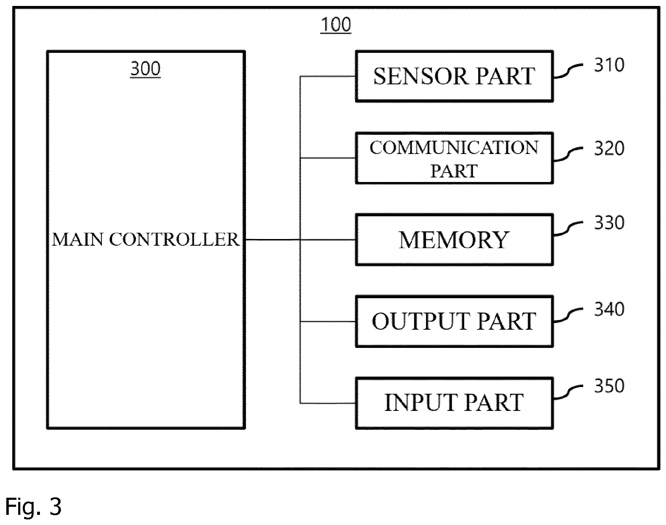

FIG. 1 is a view showing a mobile medical imaging device according to one embodiment of the present disclosure. In addition, FIG. 2 is a view showing a process of using the medical imaging device according to one embodiment of the present disclosure. In addition, FIG. 3 is a view showing a block diagram of various components that may be included in the medical imaging device according to one embodiment of the present disclosure.

Referring to FIG. 1, a mobile medical imaging device 100 of the present disclosure may include wheels and be able to move. A medical imaging device according to one embodiment may be a device that can capture an image of and/or examine a structure inside a subject (or object) based on radiation including x-rays. For example, a medical imaging device irradiates the human body with x-rays so that the x-rays penetrate the human body, scans the penetrating x-rays, and obtains an image of the inside of the human body.

Referring to FIGS. 1 to 3, the medical imaging device 100 may include a source assembly 110, a detector 120, and a main body 130. In addition, the main body 130 of the medical imaging device 100 may include a high-voltage generator (not illustrated in the drawings), a sensor part 310, a communication part 320, a memory 330, an output part 340, an input part 350, and/or a controller 300.

Referring to FIG. 2, a user may move the medical imaging device 100 to the vicinity of a hospital bed 220. The user may place the detector 120 behind the subject. Accordingly, the radiation irradiated from the source assembly 110 may pass through the subject and reach the detector 120. The detector 120 may detect the radiation passing through the subject and may convert the radiation into an electrical signal. In addition, the detector 120 may obtain a radiation image based on the electrical signal.

Referring to FIGS. 1 to 3, the high-voltage generator according to one embodiment may generate a high voltage for x-ray generation and may apply the high voltage to an x-ray source included in the source assembly. The high-voltage generator may be included in the main body 130, but is not limited thereto, and may also be included in the source assembly 110.

The source assembly 110 according to one embodiment may include the x-ray source that receives the high voltage generated by the high-voltage generator and generates x-rays. The x-ray source may include an x-ray tube, and the x-ray tube may be implemented as a diode vacuum tube consisting of a cathode and an anode. In addition, the source assembly may include a collimator that guides a path of the x-rays irradiated from the x-ray source and adjusts an x-ray irradiation region.

The detector according to one embodiment detects the x-rays irradiated from the source assembly and passing through the object. The detector may be a digital detector. The detector may be implemented using at least one of a thin film transistor (TFT), a charge coupled device (CCD), a complementary metal-oxide semiconductor (CMOS), computed radiography (CR), and a film. The detector may be included in the medical imaging device 100 or may be a separate device that can be connected to and separated from the medical imaging device 100.

The medical imaging device 100 may include the controller 300. In the present disclosure, the controller 300 may mean at least one of a main controller included in the main body 130 and a detector controller included in the detector. In the present disclosure, a controller included in the main body is referred to as "main controller," and for a controller included in another device, a device in which the controller is included is clearly indicated. For example, a detector controller is a controller included in a mobile detector and may be a controller different from the main controller 300. The main controller 300 and the detector controller are included in different devices but may be similar in that each may include at least one of a processor and a memory. At least some of the operations of the main controller may be performed by the detector controller. In addition, at least some of the operations of the detector controller may be performed by the main controller. Accordingly, in the present disclosure, at least one operation described as an operation of the main controller may be understood as being performed by the detector controller, and at least one operation described as an operation of the detector controller may be understood as being performed by the main controller.

The main controller 300 (or controller 300) may control an operation of the medical imaging device 100. For example, the medical imaging device 100 may include the main controller 300 for controlling an operation of the source assembly 110 or a wheel actuator allowing the main body 130 to travel. The main controller 300 may include one processor or may include a plurality of processors. The main controller 300 may be included in the main body 130. When the main controller 300 includes a plurality of processors, at least some of the plurality of processors may be provided at positions physically apart from the main body 130. In addition, the medical imaging device 100 is not limited thereto and may be implemented in various other ways.

According to one embodiment of the present disclosure, the main controller 300 may control an operation of the medical imaging device 100. For example, the medical imaging device 100 may include a plurality of actuators, and the medical imaging device 100 may control an operation of the medical imaging device 100 by controlling operations of the plurality of actuators. For example, the main controller 300 may control a source assembly driver for moving the source assembly 110. In addition, the main controller 300 may obtain an x-ray image by controlling the source assembly 110 to radiate x-rays and the detector 120 to receive the x-rays passing through an object.

According to one embodiment of the present disclosure, the main controller 300 may generate a medical image. For example, the main controller 300 may generate a medical image by scanning the detector irradiated with x-rays.

The medical imaging device 100 may include the sensor part 310. The sensor part 310 may obtain various information using at least one sensor. The sensor part 310 may be provided as a sensor using a means of measurement such as pressure, potential, and an optical means. For example, the sensor part 310 may include at least one of a distance measurement sensor and an encoder. In addition, the sensor may include a pressure sensor, an infrared sensor, a light emitting diode (LED) sensor, a touch sensor, or the like. However, the sensor part is not limited thereto. The sensor part may be included in at least one of the main body, the source assembly, the detector, a source assembly arm, and a detector arm.

In addition, the medical imaging device 100 may include the communication part 320. The communication part 320 may be a component allowing the medical imaging device 100 to communicate with an internal module or an external device in a wired or wireless manner. The external device may be an external server or a user terminal. The user terminal may be a personal computer (PC), a smartphone, a tablet, or a wearable device. The communication part 320 may include a wired/wireless communication module for network connection. As a wireless communication technology, for example, wireless LAN (WLAN) (Wi-Fi), wireless broadband (WiBro), World Interoperability for Microwave Access (WIMAX), High Speed Downlink Packet Access (HSDPA), etc., may be used. As a wired communication technology, for example, Digital Subscriber Line (xDSL), Fiber To The Home (FTTH), Power Line Communication (PLC), etc., may be used. In addition, a network connection part may include a short-range communication module and may transmit and receive data to and from an arbitrary device/terminal located at a short distance. For example, as a short-range communication technology, Bluetooth, Radio Frequency Identification (RFID), Infrared Data Association (IrDA), Ultra-Wideband (UWB), ZigBee, etc., may be used, but the short-range communication technology is not limited thereto.

The medical imaging device 100 may include the memory 330. The main controller 300 may execute instructions stored in the memory. The memory 330 may be included in the main controller 300 or may be present outside the main controller 300. The memory 330 may store various information related to the medical imaging device 100. For example, the memory 330 may include information related to an operation method of the source assembly 110 or may include captured images and user authentication information, but is not limited thereto.

The memory 330 may be implemented using a nonvolatile storage medium that can continuously store arbitrary data. Examples of the memory 330 may include not only a disk, an optical disk, and a magneto-optical storage device, but also a flash memory and/or a storage device based on a battery-backup memory, but the memory 330 is not limited thereto. The memory 330 may be a volatile storage device that is a main storage device directly accessed by a processor and in which stored information is instantly erased when power is turned off, such as a random access memory (RAM) including a dynamic random access memory (DRAM) and a static random access memory (SRAM), but the memory 330 is not limited thereto. The memory 330 may be operated by the main controller 300. In addition, the main controller 300 may execute instructions included in the memory 330.

In addition, the medical imaging device 100 may further include a manipulation part providing an interface for manipulation of the medical imaging device 100. The manipulation part may include the output part 340 and the input part 350.

The output part 340 may show imaging-related information such as irradiation of x-rays under control of the main controller 300 or may output sound and an image allowing a state of the main body to be checked. The output part 340 may include a speaker or a display. The output part 340 may output a medical image generated by the main controller 300. The output part 340 may output information necessary for a user to manipulate the medical imaging device 100, such as a user interface (UI), user information, or object information. Examples of the output part 340 may include a speaker, a printer, a cathode ray tube (CRT) display, a liquid crystal display (LCD), a plasma display panel (PDP), an organic light emitting diode (OLED) display, a field emission display (FED), a light emitting diode (LED) display, a vacuum fluorescent display (VFD), a digital light processing (DLP) display, a flat panel display (FPD), a 3D display, a transparent display, etc., and may include various other output devices within the scope apparent to those skilled in the art.

The medical imaging device 100 may be connected to a work station via a wire or wirelessly. The work station may be present in a space physically separated from the medical imaging device 100.

The work station may include a storage server. The storage server may store a medical image, information on an object, information on a user (medical service provider), etc. The work station may include a review device. The review device may receive a medical image from the storage server and diagnose the medical image based on a user command. The work station and the medical imaging device 100 may send, store, process, and output data according to the Digital Imaging and Communications in Medicine (DICOM) standards. In addition, the work station may include a Picture Archiving and Communication System (PACS).

The work station may include an output part, an input part, and a controller. The output part and the input part provide an interface for manipulation of the work station and the medical imaging device 100 to the user. The controller of the work station may control the work station and the medical imaging device 100.

The medical imaging device 100 may be controlled through the work station or may be controlled by the main controller 300 included in the medical imaging device 100. Accordingly, the user may control the medical imaging device 100 through the work station or may control the medical imaging device 100 through the manipulation part and the main controller 300 included in the medical imaging device 100. In other words, the user may remotely control the medical imaging device 100 through the work station or may directly control the medical imaging device 100.

The controller of the work station and the main controller 300 of the medical imaging device 100 may be separate controllers, but the present disclosure is not limited thereto. The controller of the work station and the main controller 300 of the medical imaging device 100 may be implemented as one integrated controller, and the integrated controller may be included in only one of the work station and the medical imaging device 100. In the following description, the main controller 300 may indicate the controller of the work station and/or the controller of the medical imaging device 100.

The output part and the input part of the work station and the output part 340 and the input part 350 of the medical imaging device 100 may each provide an interface for manipulation of the medical imaging device 100 to the user. The work station and the medical imaging device 100 may each include an output part and an input part, but the present disclosure is not limited thereto. An output part or an input part may be implemented in only one of the work station and the medical imaging device 100.

In the following description, the input part 350 indicates the input part of the work station and/or the input part of the medical imaging device 100, and the output part 340 indicates the output part of the work station and/or the output part of the medical imaging device 100.

The input part 350 may receive commands for manipulating the medical imaging device 100 and various information related to x-ray imaging from the user. The main controller 300 may control or manipulate the medical imaging device 100 based on the information input through the input part 350. The input part 350 may include a joystick, a keyboard, a mouse, a touchscreen, an imaging button, an unlocking button, a voice recognition device, a fingerprint recognition device, an iris recognition device, a human body motion recognition device, etc., and may include other input devices apparent to those skilled in the art.

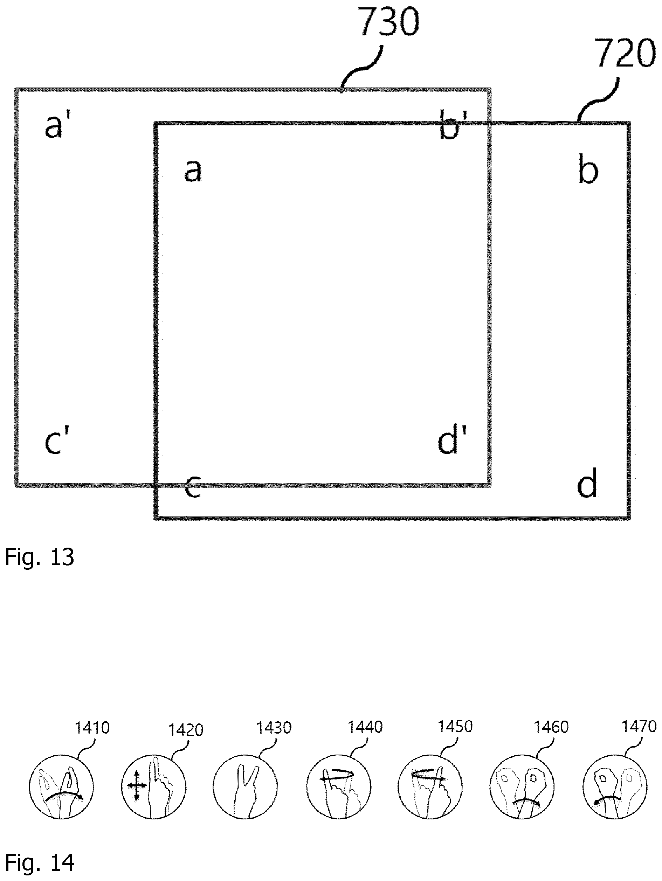

The human body motion recognition device included in the input part 350 may be implemented using at least one camera. For example, the human body motion recognition device may be implemented using a 3D camera or a depth sensor included in the source assembly 110. The main controller 300 may control an operation of the medical imaging device 100 based on the human body motion recognition device. The human body motion recognition device will be described with reference to FIG. 14.

FIG. 14 is a view for describing an operation of the medical imaging device according to one embodiment of the present disclosure.

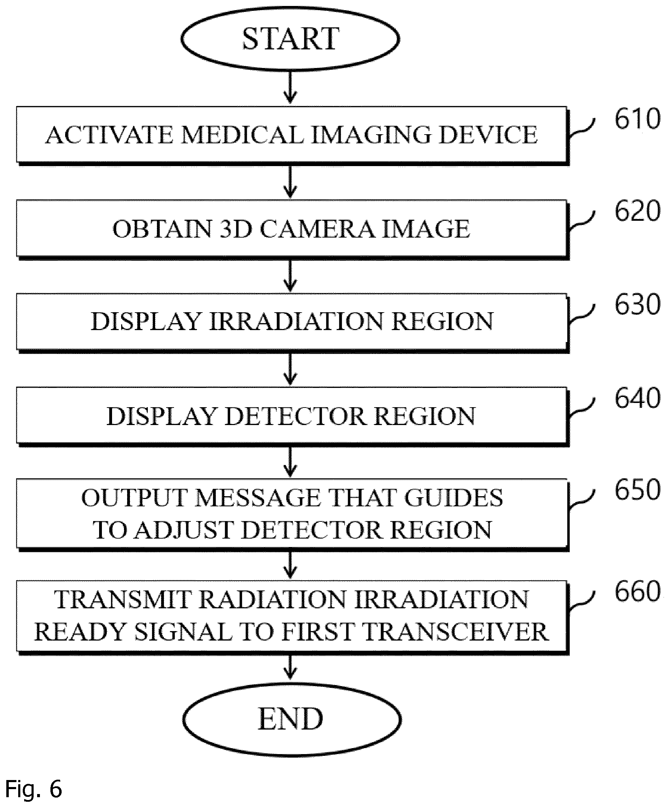

Referring to FIG. 14, a first gesture 1410 shows a motion in which a user moves his or her hand to the left and right. For example, in a space in which the human body motion recognition device recognizes a gesture, a user may make the first gesture 1410 of moving the user's hand to the left and right. The main controller 300 may perform realignment between the source assembly 110 and the detector 120 based on a signal from the human body motion recognition device indicating that the first gesture 1410 has been recognized. For example, the main controller 300 may perform a process of aligning the source assembly 110 and the detector 120 again from the beginning. For example, this process may be the same as some of the processes illustrated in FIG. 6.

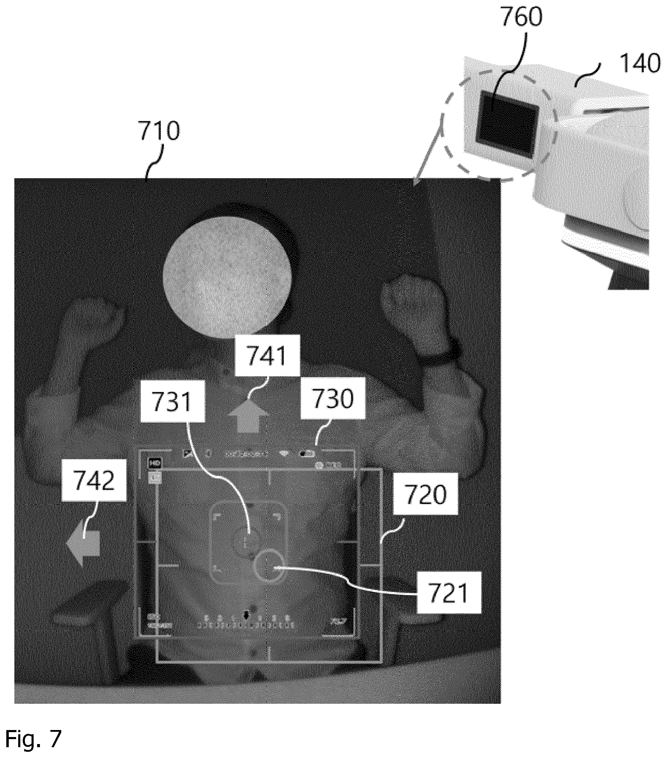

A second gesture 1420 shows a motion in which a user moves his or her hand to the left, right, front, or back while extending one finger. For example, in a space in which the human body motion recognition device recognizes a gesture, a user may make the second gesture 1420 of moving the user's hand to at least one of the left, right, front, and back while extending one of the user's fingers. The main controller 300 may control the source assembly 110 to move based on a signal from the human body motion recognition device indicating that the second gesture 1420 has been recognized. In addition, the main controller 300 may change a position of at least one of a radiation irradiation region 720 and a center 721 of the radiation irradiation region 720 shown in a 3D camera image 710 based on movement of the source assembly 110. For example, when the user moves the hand to the left while extending one finger, the source assembly may move to the left relative to the detector 120 that is fixed. In addition, due to the source assembly 110 moving to the left, the center 721 of the radiation irradiation region 720 may move to the left in the 3D camera image 710 of FIG. 7.

A third gesture 1430 shows a motion in which a user remains still while extending two fingers. For example, in a space in which the human body motion recognition device recognizes a gesture, a user may make the third gesture 1430 of remaining still while extending two of the user's fingers. The main controller 300 may determine that the alignment has been completed, based on a signal from the human body motion recognition device indicating that the third gesture 1430 has been recognized. In addition, based on the completion of the alignment, the main controller 300 may fix the source assembly 110 to prevent the source assembly 110 from moving or may prepare for radiation irradiation. Based on the completion of the alignment, the main controller 300 may transmit a signal to the detector controller for the detector 120 to be prepared to receive radiation.

A fourth gesture 1440 shows a motion in which a user rotates his or her hand clockwise while extending one finger. For example, in a space in which the human body motion recognition device is able to recognize a gesture, a user may make the fourth gesture 1440 of rotating the user's hand clockwise while extending one of the user's fingers. The main controller 300 may increase the output of the source assembly 110 based on a signal from the human body motion recognition device indicating that the fourth gesture 1440 has been recognized. However, the present disclosure is not limited thereto, and the main controller 300 may decrease the output of the source assembly 110 based on the signal from the human body motion recognition device indicating that the fourth gesture 1440 has been recognized.

A fifth gesture 1450 shows a motion in which a user rotates his or her hand counterclockwise while extending one finger. For example, in a space in which the human body motion recognition device is able to recognize a gesture, a user may make the fifth gesture 1450 of rotating the user's hand counterclockwise while extending one of the user's fingers. The main controller 300 may decrease the output of the source assembly 110 based on a signal from the human body motion recognition device indicating that the fifth gesture 1450 has been recognized. However, the present disclosure is not limited thereto, and the main controller 300 may increase the output of the source assembly 110 based on the signal from the human body motion recognition device indicating that the fifth gesture 1450 has been recognized.

A sixth gesture 1460 shows a motion in which a user forms a circle with his or her fingers and moves the hand to the right. For example, in a space in which the human body motion recognition device is able to recognize a gesture, a user may make the sixth gesture 1460 of forming a circle with the user's thumb and forefinger and moving the hand to the right. The main controller 300 may change an imaging position based on a signal from the human body motion recognition device indicating that the sixth gesture 1460 has been recognized. For example, based on the sixth gesture 1460, the main controller 300 may select a next item in a predetermined imaging position list. However, the present disclosure is not limited thereto, and based on the sixth gesture 1460, the main controller 300 may select a predetermined imaging position that corresponds to the sixth gesture 1460. For example, the predetermined imaging position may be chest. The main controller 300 may obtain predetermined radiation irradiation setting information corresponding to the selected imaging position from the memory and may apply the predetermined radiation irradiation setting information.

A seventh gesture 1470 shows a motion in which a user forms a circle with his or her fingers and moves the hand to the left. For example, in a space in which the human body motion recognition device is able to recognize a gesture, a user may make the seventh gesture 1470 of forming a circle with the user's thumb and forefinger and moving the hand to the left. The main controller 300 may change an imaging position based on a signal from the human body motion recognition device indicating that the seventh gesture 1470 has been recognized. For example, based on the seventh gesture 1470, the main controller 300 may select a previous item in a predetermined imaging position list. However, the present disclosure is not limited thereto, and based on the seventh gesture 1470, the main controller 300 may select a predetermined imaging position that corresponds to the seventh gesture 1470. For example, the predetermined imaging position may be abdomen. The main controller 300 may obtain predetermined radiation irradiation setting information corresponding to the selected imaging position from the memory and may apply the predetermined radiation irradiation setting information.

The gestures 1410 to 1470 shown in FIG. 14 and the operations of the medical imaging device 100 according thereto have been described above, but the present disclosure is not limited thereto, and one of the gestures 1410 to 1470 may correspond to one of various other operations of the medical imaging device 100. In addition, the gestures that can be recognized by the human body motion recognition device are not limited to those shown in FIG. 14.

User convenience can be improved because the medical imaging device 100 is controlled based on gestures as described above. For example, because a user can control the medical imaging device 100 at an arbitrary position without going back to the main body 130, movements of the user when capturing a medical image can be reduced. In addition, because there is no need for the user to be close to the medical imaging device 100 to input a gesture, the user's exposure to radiation can be reduced, and the user's safety can be ensured.

Referring back to FIG. 3, the user may input a command for x-ray irradiation through the input part 350, and a switch for input of such a command may be provided on the input part 350. The switch may be provided so that an irradiation command for x-ray irradiation is input when the switch is pressed at least once.

For example, the switch may have a structure in which, when a user presses the switch, a preparation command instructing that preheating for x-ray irradiation be performed is input, and in that state, when the user presses the switch deeper, an irradiation command for actual x-ray irradiation is input. When the user manipulates the switch in this way, the main controller 300 generates a signal corresponding to a command input through manipulation of the switch, that is, a preparation signal, and transmits the preparation signal to the high-voltage generator generating a high voltage for x-ray generation.

The high-voltage generator receives the preparation signal transmitted from the main controller 300, starts preheating, and when preheating is complete, transmits a preparation complete signal to the main controller 300. In addition, preparation for x-ray detection is also necessary for the detector to detect x-rays, and the main controller 300 transmits the preparation signal to the detector so that, while preheating is performed by the high-voltage generator, the detector prepares for detection of x-rays passing through an object. Upon receiving the preparation signal, the detector prepares for x-ray detection, and when preparation for detection is complete, the detector transmits a preparation-for-detection complete signal to the main controller 300.

When preheating of the high-voltage generator is complete, and preparation for x-ray detection of the detector is complete, the main controller 300 transmits an irradiation signal to the high-voltage generator, the high-voltage generator generates a high voltage and applies the high voltage to the x-ray source, and the x-ray source irradiates x-rays.

When transmitting the irradiation signal, the controller 300 may transmit a sound or light output signal to the output part 340 and may allow predetermined sound or light to be output from the output part 340 so that an object can become aware of x-ray irradiation. In addition, sound or light indicating imaging-related information other than x-ray irradiation may be output from the output part 340. The output part 340 may be included in the manipulation part but is not limited thereto, and the output part 340 or a portion of the output part 340 may be located at a different point from a point where the manipulation part is located. For example, the output part 340 or a portion of the output part 340 may be located on a wall of an imaging room where x-ray imaging is performed for an object.

The controller 300 controls positions of an x-ray irradiation part and the detector, an imaging timing, an imaging condition, and the like according to imaging conditions set by the user.

Specifically, the main controller 300 controls the high-voltage generator and the detector and controls an x-ray irradiation timing, an x-ray intensity, an x-ray irradiation region, etc., according to a command input through the input part 350. In addition, the main controller 300 adjusts the position of the detector and controls an operation timing of the detector according to predetermined imaging conditions.

In addition, the main controller 300 generates a medical image of an object using image data received through the detector. Specifically, the main controller 300 may generate a medical image of an object by receiving image data from the detector, removing noise from the image data, and adjusting a dynamic range and interleaving.

The work station may further include a communication part (not illustrated) that can be connected to a server, a medical device, a portable terminal, etc., through a network. The work station may be one of external devices.

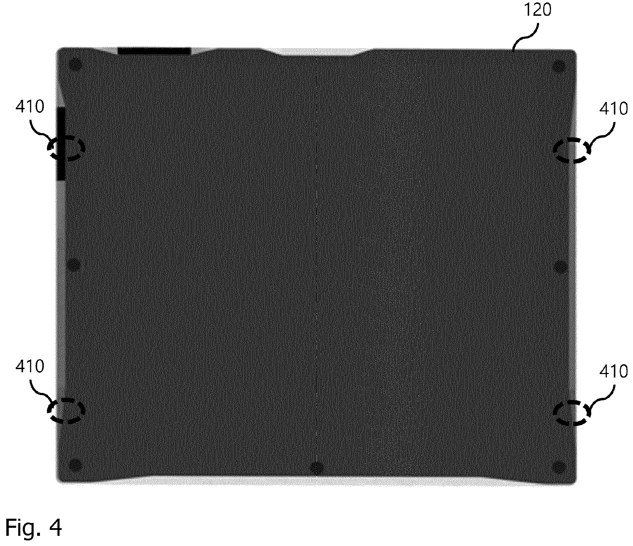

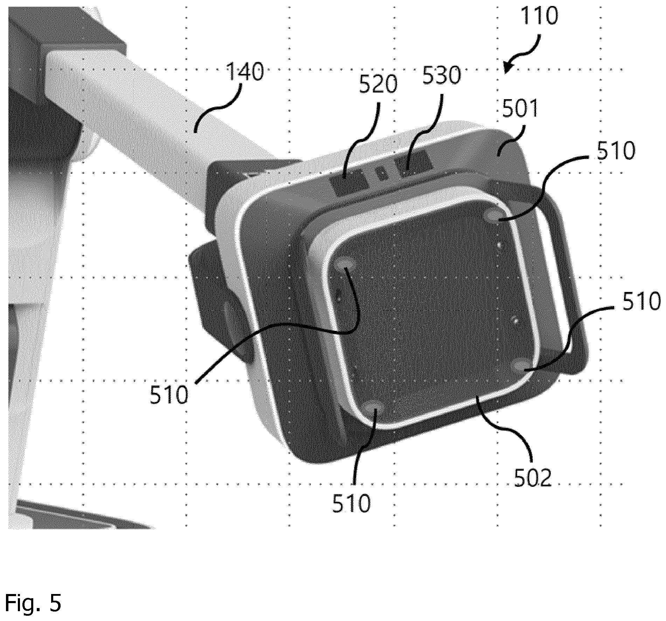

The source assembly 110 and the detector 120 will be described in detail below with reference to FIGS. 4 and 5.

FIG. 4 shows the detector according to one embodiment of the present disclosure.

The medical imaging device 100 may include the detector 120. FIG. 4 shows a rear surface of the detector 120. The rear surface of the detector 120 may be a back surface of a surface receiving radiation. The detector 120 may be separable from the medical imaging device 100. That is, the detector 120 may move independently of the medical imaging device 100. However, the detector 120 is not limited thereto and may be coupled to the medical imaging device 100.

The detector 120 may include the detector controller for controlling an operation of the detector. The detector controller may control an operation of the detector 120. The detector controller may determine radiation reception time. The radiation reception time may include a time at which radiation reception starts and a time at which radiation reception ends. The detector controller may generate a medical image based on a received signal.

The detector 120 may include a first transceiver 410 disposed at a predetermined position. FIG. 4 shows one embodiment of the first transceiver 410. However, the position of the first transceiver 410 is not limited to that shown in FIG. 4. The first transceiver 410 may be a component for wirelessly communicating with a second transceiver included in the source assembly 110. The first transceiver 410 may include a first transmitter and a first receiver. The detector 120 may include a plurality of first transceivers 410. The first transceivers 410 may be located on a surface of the detector 120 that receives radiation. The first transceivers 410 may be arranged along edges of the detector 120. The first transceivers 410 may be located on a left side surface and a right side surface of the detector 120. For example, two first transceivers 410 may be disposed on each of the left side surface and the right side surface of the detector 120. When the plurality of first transceivers 410 are arranged on the detector 120 in this way, the medical imaging device 100 can precisely align the detector 120 and the source assembly 110. However, the present disclosure is not limited thereto, and the first transceiver 410 may be located in the vicinity of a vertex of the detector 120 having a quadrangular shape.

FIG. 5 is a view for describing the source assembly according to one embodiment of the present disclosure.

FIG. 5 may be a view of a radiation irradiation surface of the source assembly 110. The source assembly 110 may be connected to the main body 130 through a source arm 140. As described above, the source assembly 110 may include an x-ray source 501 and a collimator 502. Relative to the x-ray source 501, the collimator 502 may be rotatable about an axis parallel to the longitudinal direction of the source arm 140. A radiation irradiation range may be determined by the collimator 502. Accordingly, due to the rotation of the collimator 502, the radiation irradiation range may also rotate about the axis parallel to the longitudinal direction of the source arm 140.

The source assembly 110 may include a second transceiver 510 on a front surface. The second transceiver 510 may include a second transmitter and a second receiver. The front surface of the source assembly 110 may be a surface of the source assembly 110 that is located in a direction in which the radiation exits. The front surface of the source assembly 110 may be a surface perpendicular to the direction in which the radiation exits. The second transceiver 510 may communicate with the first transceiver 410. The first transceiver 410 and the second transceiver 510 may communicate with each other using UWB.

The source assembly 110 may include a plurality of second transceivers 510. The second transceivers 510 may be disposed on edges of the front surface of the source assembly 110. When the plurality of second transceivers 510 are arranged on the source assembly 110 in this way, the medical imaging device 100 can precisely align the detector 120 and the source assembly 110.

The source assembly 110 may include a 3D camera 520. The 3D camera 520 may be a component for obtaining at least one of posture information of the detector 120 and posture information of the source assembly 110. The 3D camera 520 may include at least one of a depth measurement camera, a red-green-blue-depth (RGBD) camera, or a time-of-flight (TOF) camera. The 3D camera 520 is one type of imaging device that measures the time taken for light from the camera to reach the camera after being reflected by an object and measures a distance to the object shown in an image. The 3D camera 520 may be generally used in robotics, virtual/augmented reality, autonomous driving vehicles, and applications such as an indoor navigation.

The 3D camera 520 may use a special type of image sensor that is sensitive to both intensity and time. The 3D camera 520 may emit a modulated light signal (visible light or infrared light) and may measure the time taken for light to return to the camera after being reflected by an object. In addition, the 3D camera 520 may measure a phase shift of the modulated signal and may calculate a distance to the object. The 3D camera 520 may repeat such a process several times per second and may generate a 3D map for an image. Since the 3D camera 520 emits infrared light and detects light returning after the infrared light is reflected by an object, the 3D camera 520 may obtain an infrared image of the object. However, the present disclosure is not limited thereto, and the 3D camera 520 may emit visible light, may detect light returning after the visible light is reflected by an object, and may obtain a visible image of the object. Although description with reference to the drawings of the present disclosure is mainly given assuming that the medical imaging device 100 obtains an infrared image, the present disclosure is not limited thereto. The term "infrared image" used below may be replaced with the term "3D camera image." In addition, the term "3D camera image" is a term encompassing at least one of an infrared image and a visible image, refers to an image that may be displayed on the medical imaging device 100, and may refer to an image capturing surfaces of a subject whose image will be captured by the medical imaging device 100 and objects around the subject.

The 3D camera 520 may operate in wide-range lighting conditions and may be less affected by ambient lighting and reflection. In addition, the 3D camera 520 may have a high frame speed and may capture a fast-moving object in real time.

The source assembly 110 may additionally include a gyro sensor 530. The gyro sensor 530 may measure a posture of the source assembly 110 in a 3D space. For example, the gyro sensor 530 may measure a degree of rotation based on at least one of a first axis parallel to the ground, a second axis parallel to the ground and perpendicular to the first axis, and a third axis perpendicular to the ground. The detector 120 may also include a gyro sensor, and based on the gyro sensor of the detector 120 and the gyro sensor 530 of the source assembly 110, the medical imaging device 100 may guide a radiation irradiation direction of the source assembly 110 to be aligned to be perpendicular to a surface of the detector 120.

An operation of the main controller 300 included in the main body 130 will be described below.

FIG. 6 is a flowchart for describing an operation of the medical imaging device according to one embodiment of the present disclosure.

When an intensity of a signal from the first transceiver 410 that is received by the second transceiver 510 is higher than a predetermined threshold signal intensity, the main controller 300 may perform a step of activating the medical imaging device (610). The intensity of the signal from the first transceiver 410 that is received by the second transceiver 510 may be determined based on a distance between the second transceiver 510 and the first transceiver 410. That is, the intensity of the signal from the first transceiver 410 that is received by the second transceiver 510 may be higher with a decrease in the distance between the second transceiver 510 and the first transceiver 410. In addition, the distance between the first transceiver 410 and the second transceiver 510 may be a distance between the source assembly 110 and the detector 120. That is, the medical imaging device may be activated when the distance between the source assembly 110 and the detector 120 is within an activation threshold distance.

The first transceiver 410 and the second transceiver 510 may have to be in an activated state for the first transceiver 410 and the second transceiver 510 to communicate. More specifically, when the user has activated the mobile detector 120 by pressing a button of the mobile detector 120, the detector controller may transmit a signal to the second transceiver 510 using the first transceiver 410. The main controller 300 may activate the medical imaging device 100 when it is confirmed that the second transceiver 510 has received a signal from the detector 120. Here, activating the medical imaging device 100 may mean letting the medical imaging device 100 enter a partial sleep mode or a radiation irradiation mode from a deep sleep mode, for example. The deep sleep mode is a state in which almost all functions included in the detector are deactivated. For example, the deep sleep mode may be a state in which functions except for functions of at least one of the main controller 300 and the second transceiver 510 are deactivated. The partial sleep mode may be a state in which only some functions are deactivated. The radiation irradiation mode may be a state right before radiation irradiation or a state in which preparation for radiation irradiation is performed. In addition, the radiation irradiation mode may mean that the medical imaging device 100 displays a screen for preparation for radiation irradiation on the display.

The main controller 300 may perform the following process to activate the medical imaging device 100. The main controller 300 may perform a step of receiving identification information of the detector using the second transceiver 510. In addition, the main controller 300 may obtain feature information of the detector based on the identification information of the detector. The main controller 300 may reflect the feature information of the detector in radiation irradiation setting information. The radiation irradiation setting information may include at least one of a radiation intensity, a radiation irradiation timing, a radiation irradiation time, and a radiation irradiation range. In this way, since the medical imaging device 100 irradiates radiation optimally according to the detector, a high-quality medical image can be obtained.

Although the above description has been given from a viewpoint of the medical imaging device 100, a similar process may be performed for the detector 120.

When an intensity of a signal from the second transceiver 510 that is received by the first transceiver 410 is higher than a predetermined threshold signal intensity, the detector controller included in the detector 120 may activate the detector. More specifically, when the user has activated the medical imaging device 100 by pressing a button of the medical imaging device 100, the main controller 300 may transmit a signal to the first transceiver 410 using the second transceiver 510. The detector controller may activate the detector 120 when it is confirmed that the first transceiver 410 has received a signal from the medical imaging device 100. Here, activating the detector 120 may mean letting the detector 120 enter a partial sleep mode or a radiation reception mode from a deep sleep mode, for example. The deep sleep mode is a state in which almost all functions included in the detector are deactivated, and the partial sleep mode may be a state in which only some functions are deactivated. There may be more activated functions in the partial sleep mode than in the deep sleep mode. For example, the partial sleep mode may be a state in which components for generating a radiation image are deactivated, and other functions are activated. At this time, the activated functions may be at least one of a communication function and an input/output function. The radiation reception mode may be a state in which a radiation image can be obtained by receiving radiation. That is, when the first transceiver 410 has received an irradiation ready signal indicating that a region of the detector and an irradiation region coincide from the second transceiver 510, the detector controller may enter the radiation reception mode, and a radiation receiving element included in the detector may detect radiation and generate an electrical signal. The detector controller may detect radiation for a predetermined amount of time, may end radiation reception, and may generate a medical image based on radiation signals received up to that point.

After activating the detector, the detector controller may transmit at least one of the identification information of the detector and the feature information of the detector to the second transceiver 510 using the first transceiver 410. The main controller of the source assembly 110 may determine radiation irradiation setting information based on at least one of the identification information of the detector and the feature information of the detector. Since the medical imaging device 100 reflects individual features of the detector in obtaining a medical image, a high-quality medical image can be obtained.

The main controller 300 may perform a step of obtaining a 3D camera image of at least one of the subject and the detector based on the 3D camera 520 (620). As described above, the 3D camera 520 may be implemented to measure a distance from the 3D camera 520 to at least one image-captured object using at least one of infrared light and visible light. Accordingly, the 3D camera 520 may not only measure the distance to the image-captured object but also obtain a 3D camera image. The 3D camera image may be an image based on at least one of infrared light and visible light received by the 3D camera 520 again after being irradiated from the 3D camera 520 and being reflected from the subject and objects around the subject. That is, the subject and the objects around the subject may be shown in the 3D camera image. The surfaces of the subject and the objects around the subject may be shown in the 3D camera image. A radiation image may be different from the 3D camera image in that internal states of the subject and the objects around the subject may be shown. In addition, since the detector 120 will be placed behind the subject, the detector 120 may be included in the 3D camera image. However, the present disclosure is not limited thereto, and the detector 120 may be blocked from view by the subject and not shown in the 3D camera image. The radiation radiated from the source assembly 110 may pass through the subject and reach the detector 120. The main controller 300 may detect the detector shown in the 3D camera image using a machine learning model. In addition, even when the detector is not shown in the 3D camera image, the main controller 300 may detect the position of the detector 120 relative to the source assembly 110 based on signals of the first transceiver 410 and the second transceiver 510.

FIG. 7 will be referenced for a moment for description of step (630) and step (640).

FIG. 7 is a view for describing the medical imaging device according to one embodiment of the present disclosure.

Referring to FIGS. 6 and 7, displaying a radiation irradiation region in the 3D camera image based on state information of the source assembly 110 (630) may be performed. The state information of the source assembly 110 may be at least one of the mode of the source assembly and position information of a blade included in the collimator. For example, when the mode of the source assembly 110 is an imaging mode, the medical imaging device 100 may display the radiation irradiation region 720 in the 3D camera image 710. The 3D camera 520 may be disposed at a fixed position relative to the source assembly 110. Accordingly, in the 3D camera image obtained by the 3D camera 520, the center 721 of the radiation irradiation region 720, which will be irradiated with radiation by the source assembly 110, may be located at a fixed position. The main controller 300 may display the center 721 of the radiation irradiation region 720 at a predetermined position in the 3D camera image. However, the present disclosure is not limited thereto, and the main controller 300 may display the center 721 of the radiation irradiation region 720 based on the position information of the blade included in the collimator. Although the radiation irradiation region 720 and the center 721 of the radiation irradiation region 720 are shown simultaneously in the 3D camera image 710 of FIG. 7, the present disclosure is not limited thereto. The main controller 300 may display at least one of the radiation irradiation region 720 and the center 721 of the radiation irradiation region 720.

In addition, the state information of the source assembly 110 may include the position information of the blade included in the collimator. The blade included in the collimator may include a radiopaque material. The blade of the collimator may block a portion of radiation generated from the x-ray source and may determine a radiation irradiation region. The main controller 300 may determine at least one of the size and position of the radiation irradiation region 720 displayed in the 3D camera image 710 based on the state information of the source assembly 110 including the position information of the blade included in the collimator. In addition, the main controller 300 may display the center 721 of the radiation irradiation region 720 based on the position of the radiation irradiation region 720. The main controller 300 may display the radiation irradiation region 720 in the 3D camera image 710 based on at least one of the size and position of the radiation irradiation region 720.

In addition, the main controller 300 may perform a step of displaying a detector region 730 in the 3D camera image 710 (640). The main controller 300 may perform a process for locating the detector region 730, and the process for locating the detector region 730 will be described below. The main controller 300 may display at least one of the detector region 730 and a center 731 of the detector region 730 in the 3D camera image 710.

The medical imaging device 100 may include the output part 340. The output part 340 may include a display 760. The display 760 may be included in at least one of the main body 130 and the source arm 140. The medical imaging device 100 may display at least one of the 3D camera image, the radiation irradiation region, and the detector region on the display 760.

Referring back to FIG. 6, the main controller 300 may perform a step of outputting a message guiding to adjust the irradiation region and the detector region 730 based on alignment information (650). The main controller 300 may determine alignment information related to at least one of a direction in which the source assembly 110 has to move, a distance the source assembly 110 has to move, and an angle at which the source assembly 110 has to move, in order to align the detector 120 and the source assembly 110 based on at least one of the 3D camera, the first transceiver 410, and the second transceiver 510. The main controller 300 may obtain the alignment information to perform the outputting of the message (650). The alignment information may be information for aligning the source assembly 110 and the detector 120. Here, aligning the detector 120 and the source assembly 110 may mean letting the irradiation region and the detector region coincide or letting a line connecting the center of the detector and the center of the source assembly 110 be parallel to a radiation irradiation direction. The alignment information may include at least one of first alignment information, second alignment information, and third alignment information.

The main controller 300 may obtain at least one of the posture information of the detector 120 and the posture information of the source assembly 110 based on the first transceiver 410 and the second transceiver 510. The main controller 300 may further use the 3D camera to obtain at least one of the posture information of the detector 120 and the posture information of the source assembly 110. Alternatively, the main controller 300 may further use the 3D camera to revise at least one of the posture information of the detector 120 and the posture information of the source assembly 110.

The main controller 300 may perform a step of obtaining information for adjusting an angle of the source assembly 110 so that a radiation irradiation direction of the source assembly 110 becomes perpendicular to a radiation reception surface of the detector 120 based on at least one of the posture information of the detector 120 and the posture information of the source assembly 110. The posture information of the detector 120 may be obtained based on at least one of the gyro sensor and the first transceiver 410 included in the detector 120. The detector 120 may directly measure the posture information based on the gyro sensor or may indirectly measure the posture information by triangulation using the first transceiver 410 and the second transceiver 510. The posture information of the detector 120 may include at least one of a degree of inclination (slope) of the detector 120 relative to the source assembly 110 or the ground and a distance from the detector 120 to the source assembly 110 or the ground. The posture information of the detector 120 may include a degree of rotation of the detector 120 based on at least one of a first axis parallel to the ground, a second axis parallel to the ground and perpendicular to the first axis, and a third axis perpendicular to the ground. The posture information of the detector 120 may include a distance from one point of the source assembly 110 to one point of the detector 120. The posture information of the detector 120 may include coordinates from one of one point of the source assembly 110 and one point of the main body 130 to one point of the detector 120. The one point of the source assembly 110 may be one of the center of the source assembly 110 and the center of a front surface of the source assembly 110. In addition, the one point of the detector 120 may be the center of the radiation reception surface of the detector 120. However, the one point of the main body 130, the one point of the source assembly 110, and the one point of the detector 120 are not limited to the above description as long as they are points included in the main body 130, the source assembly 110, and the detector 120, respectively. The posture information of the detector 120 may include a slope of the radiation reception surface of the detector 120 relative to the front surface of the source assembly 110.

The main controller 300 included in the medical imaging device 100 may obtain the posture information of the source assembly 110 based on at least one of the gyro sensor and the second transceiver 510. The posture information of the source assembly 110 may include at least one of a degree of inclination (slope) of the source assembly 110 relative to the detector 120 or the ground and a distance from the source assembly 110 to the detector 120 or the ground. The main controller 300 may directly measure the posture information of the source assembly 110 based on the gyro sensor or may indirectly measure the posture information of the source assembly 110 by triangulation using the first transceiver 410 and the second transceiver 510. The posture information of the source assembly 110 may include a degree of rotation of the source assembly 110 based on at least one of the first axis, the second axis, and the third axis. The posture information of the source assembly 110 may include a distance from one of one point of the detector 120 and one point of the main body 130 to one point of the source assembly 110. The posture information of the source assembly 110 may include coordinates from one point of the source assembly 110 to one point of the detector 120. The posture information of the source assembly 110 may include a slope of the front surface of the source assembly 110 relative to the radiation reception surface of the detector 120.

The process of obtaining posture information based on the first transceiver and the second transceiver has been described above, but the present disclosure is not limited thereto. The main controller 300 may obtain at least one of the posture information of the source assembly 110 and the posture information of the detector 120 based on the 3D camera 520. That is, the main controller 300 may obtain at least one of the posture information of the source assembly 110 and the posture information of the detector 120 based on the 3D camera image obtained by the 3D camera 520. The 3D camera 520 included in the source assembly 110 may capture an image of the detector 120. The main controller 300 may measure a depth (distance) from the front surface of the source assembly 110 to each point included in the radiation reception surface of the detector based on the 3D camera image obtained by the 3D camera. The main controller 300 may output at least one of the posture information of the source assembly 110 and the posture information of the detector 120. That is, the main controller 300 may control the output part 340 to output at least one of the posture information of the source assembly 110 and the posture information of the detector 120.

According to one embodiment of the present disclosure, when performing step (650), the main controller 300 may determine alignment information related to at least one of a direction in which the source assembly 110 has to move, a distance the source assembly 110 has to move, and an angle at which the source assembly 110 has to move based on at least one of the posture information of the detector 120 and the posture information of the source assembly 110, in order to align the detector and the source assembly. Although description is given herein based on alignment information related to movement of the source assembly, the present disclosure is not limited thereto, and description may be the same even when the alignment information related to movement of the source assembly is replaced with alignment information related to movement of the detector. The alignment information may include first alignment information, second alignment information, and third alignment information. Here, aligning the detector 120 and the source assembly 110 may mean letting the irradiation region and the detector region coincide or letting a line connecting the center of the detector and the center of the source assembly 110 be parallel to a radiation irradiation direction.

According to the present disclosure, the main controller 300 may obtain at least one of pre-posture information and pre-alignment information using the first transceiver 410 and the second transceiver 510. In addition, the main controller 300 may determine at least one of post-posture information and post-alignment information based on the 3D camera image of the 3D camera 520. The pre-posture information may include at least one of pre-detector posture information and pre-source assembly posture information. The post-posture information may include at least one of post-detector posture information and post-source assembly posture information. The pre-alignment information may include at least one of pre-first alignment information, pre-second alignment information, and pre-third alignment information. The post-alignment information may include at least one of post-first alignment information, post-second alignment information, and post-third alignment information. Although it has been described above that the post-posture information and the post-alignment information are obtained based on the 3D camera 520, the present disclosure is not limited thereto, and the pre-posture information and the pre-alignment information may be obtained based on the 3D camera 520. Such a process will be described in more detail below.

According to one embodiment of the present disclosure, the main controller 300 may independently determine posture information using the 3D camera 520. Here, the posture information may include at least one of the posture information of the source assembly 110 and the posture information of the detector 120. In addition, the first transceiver 410 and the second transceiver 510 may be used first to determine posture information and then the 3D camera 520 may be used subsidiarily to correct the posture information. However, the present disclosure is not limited thereto, and the 3D camera 520 may be used first to determine posture information and then the first transceiver 410 and the second transceiver may be used subsidiarily to correct the posture information.

For example, the main controller 300 may determine pre-posture information using the first transceiver 410 and the second transceiver. Next, the main controller 300 may determine post-posture information based on the 3D camera image of the 3D camera 520. The pre-posture information may include pre-detector posture information and pre-source assembly posture information. The post-posture information may include post-detector posture information and post-source assembly posture information.

The main controller 300 may determine final posture information based on the pre-posture information and the post-posture information. More specifically, the main controller 300 may determine that the pre-posture information should be corrected when a difference in size between the pre-posture information and the post-posture information is greater than predetermined threshold alignment information. Here, the size of the posture information may be one of the size of a distance and the size of an angle. When it is determined that correction is necessary, the main controller 300 may obtain a weighted average of the pre-posture information and the post-posture information to determine the final posture information.

Final posture information = W1 * Pre-posture information + W2 * Post-posture information

Here, W1 and W2 are predetermined constants, and W1+W2 may be 1. In addition, W1 may be greater than or equal to W2. The main controller 300 may determine alignment information based on the final posture information.

According to various embodiments of the present disclosure, the main controller 300 may determine alignment information using the 3D camera 520. In addition, the first transceiver 410 and the second transceiver 510 may be used first to determine alignment information and then the 3D camera 520 may be used subsidiarily to correct the alignment information. However, the present disclosure is not limited thereto, and the 3D camera 520 may be used first to determine alignment information and then the first transceiver 410 and the second transceiver may be used subsidiarily to correct the alignment information.

For example, the main controller 300 may obtain pre-alignment information using the first transceiver 410 and the second transceiver 510. The pre-alignment information may be information obtained without help of the 3D camera 520. The pre-alignment information may be uncorrected alignment information. Next, the main controller 300 may obtain post-alignment information based on the 3D camera image of the 3D camera 520.

The main controller 300 may determine final alignment information based on the pre-alignment information and the post-alignment information. The final alignment information is corrected alignment information and may include final first alignment information, final second alignment information, and final third alignment information. More specifically, the main controller 300 may determine that the pre-alignment information should be corrected when a difference in size between the pre-alignment information and the post-alignment information is greater than predetermined threshold alignment information. Here, the size of the alignment information may be one of the size of a distance and the size of an angle. When it is determined that correction is necessary, the main controller 300 may obtain a weighted average of the pre-alignment information and the post-alignment information to determine the final alignment information.

Final alignment information = W3 * Pre-alignment information + W4 * Post-alignment information

Here, W3 and W4 are predetermined constants, and W3+W4 may be 1. In addition, W3 may be greater than or equal to W4.

The main controller 300 may determine that the pre-alignment information does not need correction when the difference in size between the pre-alignment information and the post-alignment information is smaller than or equal to the predetermined threshold alignment information. That is, the main controller 300 may determine the pre-alignment information as the final alignment information. The main controller 300 may guide movement of the source assembly 110 or movement of the detector 120 based on the final alignment information.

According to one embodiment of the present disclosure, the main controller 300 may obtain information for adjusting an angle of the source assembly 110 so that the radiation irradiation direction of the source assembly 110 becomes perpendicular to the radiation reception surface of the detector 120 based on a differential between the posture information of the detector 120 and the posture information of the source assembly 110. Although description has been given above based on the first axis, the second axis, and the third axis, another coordinate system may be used. For example, an axis parallel or perpendicular to a predetermined surface of at least one of the source assembly 110 and the detector 120 about a predetermined point of at least one of the source assembly 110 and the detector 120 may be used. In addition, the center of the coordinate system may be included in one of the source assembly 110 and the detector 120. That is, the center of the coordinate system may be movable based on one of the source assembly 110 and the detector 120. According to various embodiments of the present disclosure, for the radiation irradiation direction of the source assembly 110 to become perpendicular to the radiation reception surface of the detector 120, an angle of rotation that is necessary based on a roll axis parallel to the longitudinal direction of the source arm 140, a pitch axis perpendicular to the roll axis, and a yaw axis perpendicular to the roll axis and the pitch axis may be displayed on the output part. The direction of rotation and the angle of rotation of the source assembly 110 for the radiation irradiation direction of the source assembly 110 to become perpendicular to the radiation reception surface of the detector 120 may be referred to as "third alignment information."

The main controller 300 may perform a step of obtaining first alignment information of the source assembly 110 for adjusting the center 721 of the radiation irradiation region 720 of the source assembly 110 to coincide with the center 731 of the detector region 730 based on the posture information of the detector 120. According to various embodiments of the present disclosure, the main controller 300 may perform a step of obtaining first alignment information of the source assembly 110 for adjusting the radiation irradiation region 720 of the source assembly 110 to coincide with the detector region 730 based on the posture information of the detector 120. In addition, according to various embodiments of the present disclosure, the main controller 300 may perform a step of obtaining first alignment information of the source assembly 110 for adjusting the radiation irradiation region 720 of the source assembly 110 to include the detector region 730 based on the posture information of the detector 120. In the present disclosure, the expression "adjusting the center 721 of the radiation irradiation region 720 to coincide with the center 731 of the detector region 730" is used, but this expression may be replaced with the expression "adjusting the radiation irradiation region 720 to include the detector region 730" in all cases.

The first alignment information is information including the direction and distance of movement of at least one of the detector 120 and the source assembly 110 that is necessary for the detector 120 and the source assembly 110 to be aligned. When movement is performed according to the first alignment information, the line connecting the center of the detector 120 and the center of the radiation irradiation surface of the source assembly 110 may be parallel to the radiation irradiation direction. That is, the detector 120 and the source assembly 110 may be aligned. The first alignment information may include information of movement in a direction parallel to the radiation reception surface of the detector 120. Alternatively, the first alignment information may include information of movement in a direction parallel to a surface perpendicular to the radiation irradiation direction of the source assembly 110.

The posture information of the detector 120 may be the position of the detector 120 relative to the source assembly 110. The posture information of the detector 120 may be a 3D position from a predetermined point included in the source assembly 110 to a predetermined point included in the detector 120. The posture information of the detector 120 may include a source-to-image distance (SID). The main controller 300 may obtain first alignment information according to which the source assembly 110 has to move on a plane parallel to the front surface of the source assembly 110. The front surface of the source assembly 110 may be a surface of the source assembly 110 that is located in a direction in which the radiation exits. The front surface of the source assembly 110 may be a surface perpendicular to the direction in which the radiation exits. The front surface of the source assembly 110 may be controlled to be parallel to a surface of the detector.

Referring to FIG. 7, the main controller 300 may display, on the display, an arrow 741 indicating that upward or downward movement is necessary on the plane parallel to the front surface of the source assembly 110. The arrow 741 may be displayed to be longer or thicker with an increase in the distance that the source assembly 110 has to move. In addition, the distance that the source assembly 110 has to move may be displayed around the arrow 741. However, the present disclosure is not limited thereto. In addition, the main controller 300 may display an arrow 742 indicating that rightward or leftward movement is necessary on the plane parallel to the front surface of the source assembly 110. The arrow 742 may be displayed to be longer or thicker with an increase in the distance that the source assembly 110 has to move. In addition, the distance that the source assembly 110 has to move may be displayed around the arrow 742. However, the present disclosure is not limited thereto. The distance that the source assembly 110 has to move may be obtained based on the first alignment information.

According to various embodiments of the present disclosure, the main controller 300 may display the center 721 of the radiation irradiation region 720 and the center 731 of the detector region 730 on the display. The center 721 of the radiation irradiation region 720 and the center 731 of the detector region 730 may be displayed in various colors. The center 721 of the radiation irradiation region 720 and the center 731 of the detector region 730 may be displayed in different colors. However, the present disclosure is not limited thereto, and the center 721 of the radiation irradiation region 720 and the center 731 of the detector region 730 may be displayed in the same color. For example, the center 721 of the radiation irradiation region 720 may be displayed in white.

The center 721 of the radiation irradiation region 720 and the center 731 of the detector region 730 may have various shapes. The center 721 of the radiation irradiation region 720 and the center 731 of the detector region 730 may have the same shape, but the present disclosure is not limited thereto, and the center 721 of the radiation irradiation region 720 and the center 731 of the detector region 730 may have different shapes. For example, the center 721 of the radiation irradiation region 720 and the center 731 of the detector region 730 may each have a circular shape.

The main controller 300 may obtain at least one of the direction and distance for the center 721 of the radiation irradiation region 720 to reach the center 731 of the detector region 730 as the first alignment information. Based on the first alignment information, the main controller 300 may display at least one of the direction and distance for the center 721 of the radiation irradiation region 720 to reach the center 731 of the detector region 730 on the display. The user may, while looking at the center 721 of the radiation irradiation region 720 and the center 731 of the detector region 730, move the source assembly 110 so that the center 721 of the radiation irradiation region 720 and the center 731 of the detector region 730 coincide. The medical imaging device 100 of the present disclosure may display the center 721 of the radiation irradiation region 720 and the center 731 of the detector region 730 to allow the user to promptly align the source assembly 110 and the detector 120.

The main controller 300 may display, on the display, third alignment information for the radiation reception surface of the detector 120 and the radiation irradiation direction of the source assembly 110 to become perpendicular . The third alignment information may include at least one of the direction and angle of rotation of the source assembly 110 that is necessary for the radiation reception surface of the detector 120 and the radiation irradiation direction of the source assembly 110 to become perpendicular. The third alignment information may include at least one of the direction and angle of rotation about at least one of the roll axis, the pitch axis, and the yaw axis. The medical imaging device 100 of the present disclosure may display the third alignment information to allow the user to promptly align the radiation reception surface of the detector 120 and the radiation irradiation direction to become perpendicular.

The main controller 300 may perform a step of obtaining second alignment information of the source assembly 110 for adjusting a distance between the source assembly 110 and the detector 120 based on at least one of the posture information of the detector 120 and a source-to-object distance (SOD). The direction of the second alignment information may be perpendicular to the direction of the first alignment information. The direction of the second alignment information may be a direction perpendicular to the front surface of the source assembly 110. The direction of the second alignment information may be a direction parallel to the radiation irradiation direction. The main controller 300 may perform a step of obtaining second alignment information based on a source-to-image distance (SID) included in the posture information of the detector 120. The main controller 300 may perform a step of obtaining second alignment information based on the source-to-object distance (SOD).

For example, when the measured SID is different from a predetermined reference SID, the main controller 300 may obtain second alignment information for the measured SID to be the same as the reference SID. The second alignment information may include at least one of the direction and distance of movement of the source assembly 110 that is necessary for the measured SID to be the same as the reference SID. In addition, for example, when the measured SOD is different from a predetermined reference SOD, the main controller 300 may obtain second alignment information for the measured SOD to be the same as the reference SOD. The second alignment information may include at least one of the direction and distance of movement of the source assembly 110 that is necessary for the measured SOD to be the same as the reference SOD.

In the above, the step of obtaining the second alignment information has been described after describing the step of obtaining the first alignment information. However, the present disclosure is not limited thereto, and the step of obtaining the first alignment information and the step of obtaining the second alignment information may be performed simultaneously. In addition, according to various embodiments of the present disclosure, the step of obtaining the second alignment information may be performed after the step of obtaining the first alignment information, or the step of obtaining the second alignment information may be performed prior to the step of obtaining the first alignment information.

When the detector region 730 and the irradiation region 720 coincide, the controller 300 may output a signal indicating that the detector region 730 and the irradiation region 720 coincide. The controller 300 may use alignment information to allow the detector region 730 and the irradiation region 720 to coincide. The controller 300 may control the output part 340 to output the signal indicating the coincidence. The controller 300 may output the signal indicating the coincidence prior to or while performing step (660). The outputting of the signal indicating the coincidence may be performed prior to, after, or while performing step (650). "The detector region 730 and the irradiation region 720 coincide" may mean that the detector and the source assembly are aligned. The controller 300 may use at least one of a display and a speaker to output the fact that the detector region 730 and the irradiation region 720 coincide. "The detector region 730 and the irradiation region 720 coincide" may mean that edges of the detector region 730 and the irradiation region 720 coincide with each other. However, the present disclosure is not limited thereto, and "the detector region 730 and the irradiation region 720 coincide" may indicate that the center 731 of the detector region 730 and the center 721 of the irradiation region 720 coincide. In the present disclosure, the expression "the center 731 of the detector region 730 and the center 721 of the irradiation region 720 coincide" may be replaced with the expression "edges of the detector region 730 and the irradiation region 720 coincide". According to the medical imaging device of the present disclosure, the user may easily recognize that the detector region 730 and the irradiation region 720 coincide and may easily recognize an optimal point in time to perform imaging.

When the center 721 of the radiation irradiation region 720 does not coincide with the center 731 of the detector region 730 at the current position of the source assembly 110, the controller 300 may perform a step of displaying the radiation irradiation region 720 and the detector region 730 at the current position of the source assembly 110 in different colors in the 3D camera image 710. In addition, when the center of the detector region 730 and the center of the radiation irradiation region 720 coincide, the controller 300 may perform a step of displaying the radiation irradiation region 720 and the detector region 730 in the same color in the 3D camera image 710. Accordingly, the user may easily recognize that the center of the radiation irradiation region 720 and the center of the detector region 730 coincide.

Referring to FIG. 6, when the center 731 of the detector region 730 and the center 721 of the radiation irradiation region 720 coincide, the main controller 300 may perform a step of transmitting a radiation irradiation ready signal to the first transceiver 410 of the detector 120 using the second transceiver 510 (660). The detector 120 may enter the radiation reception mode based on the radiation irradiation ready signal. The detector 120 may start receiving radiation based on the radiation irradiation ready signal. When the first transceiver 410 receives a signal indicating an end of radiation irradiation from the second transceiver 510 after the detector 120 enters the radiation reception mode, a readout circuit part included in the detector may process an electrical signal generated by the radiation receiving element, and radiation image information may be obtained. In this way, since the detector 120 starts radiation reception and ends radiation reception by communicating with the source assembly 110, a high-quality medical image with small noise can be obtained. In addition, since the detector 120 ends radiation reception by communicating with the source assembly 110, the medical imaging device 100 can shorten the amount of time during which the subject is unnecessarily exposed to radiation and can reduce the subject's exposure to radiation. In addition, the medical imaging device 100 can obtain a medical image with sufficient quality while reducing the subject's exposure to radiation. However, the present disclosure is not limited thereto, and the detector controller may receive radiation using the radiation receiving element for a predetermined amount of time without receiving the signal indicating the end of radiation irradiation.

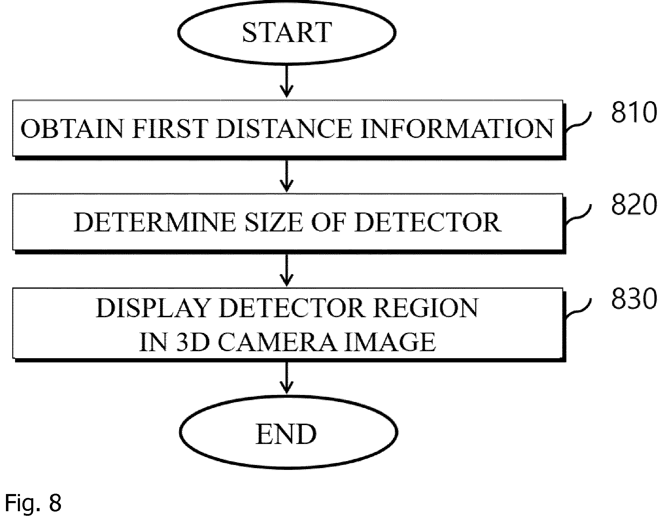

FIG. 8 is a flowchart for describing a method of obtaining the detector region from the 3D camera image according to one embodiment of the present disclosure.

The main controller 300 may perform the following operation to display the detector region 730 in the 3D camera image 710.

The main controller 300 may perform a step of obtaining first distance information between the second transceiver 510 and the first transceiver 410 based on a signal between the second transceiver 510 and the first transceiver 410 (810). The first distance information may be a distance between the first transceiver 410 and the second transceiver 510, but may also include an SID, which is a distance between the source assembly 110 and the detector 120. According to various embodiments of the present disclosure, the main controller 300 may obtain the first distance information between the second transceiver 510 and the first transceiver 410 and then may determine the SID based on the first distance information. More specifically, the main controller 300 may specify the position of the first transceiver 410 relative to the plurality of second transceivers 510 in a 3D space using triangulation. The main controller 300 may determine the position of the detector in the 3D space by determining the position of each of the plurality of first transceivers 410 included in the detector 120.

The main controller 300 may perform a step of determining the size of the detector in the 3D camera image based on at least one of the position of the detector, the first distance information, and the SID in the 3D space (820). Here, the size of the detector may be the size of the detector shown in the 3D camera image. The main controller 300 may pre-store the size of the detector according to at least one of the position of the detector, the first distance information, and the SID in the 3D space as a detector size table or a detector size function. The main controller 300 may determine the size of the detector according to at least one of the position of the detector, the first distance information, and the SID in the 3D space based on at least one of the detector size table and the detector size function. The size of the detector may decrease with an increase in the distance between the detector 120 and the source assembly 110.

The main controller 300 may determine the form of the detector 120 in the 3D camera image 710 based on at least one of the position of the detector, the first distance information, and the SID in the 3D space. The form of the detector 120 may not be rectangular when the radiation reception surface of the detector is tilted relative to the radiation irradiation surface of the source assembly 110.

The main controller 300 may perform a step of displaying the detector region 730 in the 3D camera image based on at least one of the first distance information, the SID, and the size of the detector (830). The 3D camera image of the main controller 300 may be obtained by the 3D camera 520. The main controller 300 may obtain not only the 3D camera image but also a distance to each point shown in the image, using the 3D camera 520. The main controller 300 may determine a set of points to which a distance obtained using the 3D camera 520 corresponds to one of the first distance information and the SID as the detector region 730. In addition, the main controller 300 may determine only a region corresponding to at least one of the size of the detector and the form of the detector in the set of the points corresponding to one of the first distance information and the SID as the detector region 730.

However, the present disclosure is not limited thereto, and the main controller 300 may determine the detector region 730 based on an image processing algorithm. For example, the main controller 300 may perform a step of obtaining the detector region from the 3D camera image obtained by the 3D camera 520. That is, the main controller 300 may determine the detector region 730 in the 3D camera image based on an object recognition algorithm. The object recognition algorithm may be a machine learning model for determining the detector region 730. The machine learning model may determine the detector region 730 even when the detector region 730 is partially hidden by a subject 210 in the 3D camera image. In addition, the main controller 300 may obtain second distance information from the 3D camera 520 to at least one image-captured object included in the 3D camera image. The second distance information may be a distance from the 3D camera 520 or the source assembly 110 to each point shown in the 3D camera image. The main controller 300 may obtain the SID from the source assembly to the detector based on the detector region 730 and the second distance information.

The main controller 300 may change an algorithm for checking the detector region 730 based on the 3D camera image. According to various embodiments of the present disclosure, when an exterior of at least a portion of the detector 120 is checked from the 3D camera image 710, the main controller 300 may determine the detector region 730 using at least one of 3D camera, the gyro sensor of the source assembly 110 and the gyro sensor of the detector 120. For example, the main controller 300 may determine the detector region 730 through image processing of the 3D camera image based on the 3D camera 520. Even when the detector is shown in the 3D camera image 710, it is not likely that the entire detector is shown, and thus the main controller 300 may obtain the posture of the detector relative to the 3D camera image 710 using at least one of the gyro sensor of the source assembly 110 and the gyro sensor of the detector 120 to obtain the posture of the detector according to the posture of the source assembly. As described above, the posture of the detector may be a degree to which the detector is tilted. The main controller 300 may display a tilted detector region 730 in the 3D camera image 710 based on the posture of the detector.

According to various embodiments of the present disclosure, when the exterior of the detector 120 is not checked from the 3D camera image 710, the main controller 300 may use at least one of the 3D camera, the gyro sensor of the source assembly 110, the gyro sensor of the detector 120, the first transceiver 410, and the second transceiver 510. Since the process of using the 3D camera, the gyro sensor of the source assembly 110, and the gyro sensor of the detector 120 has been described above, only the process of using the first transceiver 410 and the second transceiver 510 will be described. The main controller 300 may obtain the position of the first transceiver 410 relative to the plurality of second transceivers 510 using triangulation. The main controller 300 may determine the position of the detector in the 3D space by obtaining positions of the plurality of first transceivers 410 included in the detector 120. The main controller 300 may obtain at least one of the distance from the source assembly 110 to the detector and the posture of the detector relative to the source assembly 110 based on the position of the detector in the 3D space. The main controller 300 may determine the detector region 730 in the 3D camera image 710 based on the position of the detector in the 3D space.

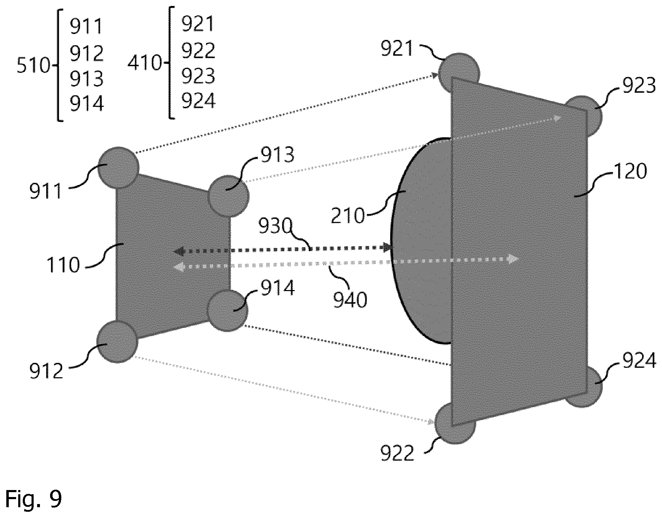

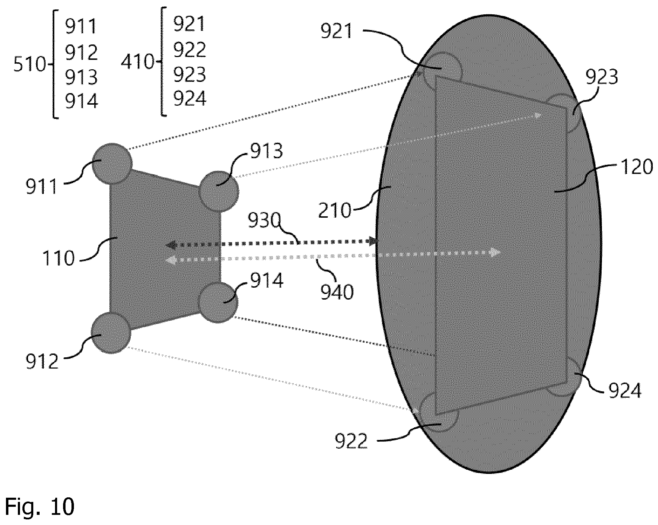

FIG. 9 is a view for describing an operation of the medical imaging device according to one embodiment of the present disclosure. FIG. 10 is a view for describing an operation of the medical imaging device according to one embodiment of the present disclosure.

Referring to FIG. 9, the detector 120 may include a plurality of first transceivers 921, 922, 923, and 924. In addition, the source assembly 110 may include a plurality of second transceivers 911, 912, 913, and 914. More specifically, the plurality of first transceivers may include a first-first transceiver 921, a first-second transceiver 922, a first-third transceiver 923, and a first-fourth transceiver 924. In addition, the plurality of second transceivers may include a second-first transceiver 911, a second-second transceiver 912, a second-third transceiver 913, and a second-fourth transceiver 914.

The main controller 300 may obtain the position of at least one of the plurality of first transceivers 921, 922, 923, and 924 by triangulation using at least three of the plurality of second transceivers 911, 912, 913, and 914. In addition, the detector controller may obtain the position of at least one of the plurality of second transceivers 911, 912, 913, and 914 by triangulation using at least three of the plurality of first transceivers 921, 922, 923, and 924. The detector controller may obtain the posture (slope) of the detector 120 relative to the source assembly 110 based on the position of at least one of the plurality of second transceivers 911, 912, 913, and 914.

According to various embodiments of the present disclosure, the detector controller may sensitively adjust a sensor at a point on the radiation reception surface of the detector that is far from the source assembly 110. Alternatively, the detector controller may insensitively adjust a sensor at a point on the radiation reception surface of the detector that is close to the source assembly 110. In addition, the detector controller or the main controller 300 may perform image processing for increasing sharpness of a medical image based on a distance between each point of radiation reception of the detector and the source assembly 110. Accordingly, a medical image may be sharp even when the surface of the detector 120 is not perpendicular to the radiation irradiation direction of the source assembly 110.

The main controller 300 may obtain a distance from one of the plurality of second transceivers to each of the plurality of first transceivers. For example, the main controller 300 may obtain a distance from the second-first transceiver 911 included in the second transceivers to each of the first-first transceiver 921, the first-second transceiver 922, the first-third transceiver 923, and the first-fourth transceiver 924. The times of the plurality of second transceivers and the plurality of first transceivers may be synchronized. When transmitting a signal to the second-first transceiver 911, the first-first transceiver 921, the first-second transceiver 922, the first-third transceiver 923, and the first-fourth transceiver 924 may also send a transmission time. The main controller 300 may obtain the distance from the second-first transceiver 911 to each of the first-first transceiver 921, the first-second transceiver 922, the first-third transceiver 923, and the first-fourth transceiver 924 based on the time at which the signal is transmitted from the first-first transceiver 921, the first-second transceiver 922, the first-third transceiver 923, and the first-fourth transceiver 924 and the time at which the signal is received by the second-first transceiver 911. In addition, the same process may be performed for the second-second transceiver 912, the second-third transceiver 913, and the second-fourth transceiver 914.

The controller 300 may match the plurality of first transceivers and the plurality of second transceivers one-to-one by matching one of the plurality of first transceivers to one of the plurality of second transceivers that is the closest thereto. The main controller 300 may match the first-first transceiver 921, which is the closest to the second-first transceiver 911, to the second-first transceiver 911. Likewise, the main controller 300 may match the first-second transceiver 922, which is the closest to the second-second transceiver 912, to the second-second transceiver 912. The main controller 300 may match the first-third transceiver 923, which is the closest to the second-third transceiver 913, to the second-third transceiver 913. The main controller 300 may match the first-fourth transceiver 924, which is the closest to the second-fourth transceiver 914, to the second-fourth transceiver 914.

When the positions of the source assembly 110 and the detector 120 are completely misaligned, in some cases, it may be difficult to match the plurality of first transceivers and the plurality of second transceivers one-to-one using the closest distance. In such cases, the medical imaging device 100 may output a message that instructs to adjust the source assembly 110 to face the detector 120 or be parallel thereto.

However, the present disclosure is not limited thereto, and the following process may be performed. The main controller 300 may obtain sixteen pieces of distance information according to sixteen combinations of the four first transceivers 410 and the four second transceivers 510. When the plurality of first transceivers and the plurality of second transceivers are not matched one-to-one, the main controller 300 may remove a combination corresponding to the shortest distance information among the sixteen pieces of distance information from the sixteen combinations. By matching each of the plurality of second transceivers 510 to one of the plurality of first transceivers 410 that is the closest thereto in the remaining fifteen combinations, the main controller 300 may match the plurality of second transceivers 510 and the plurality of first transceivers 410 one-to-one. When the plurality of first transceivers and the plurality of second transceivers are still not matched one-to-one, the main controller 300 may remove a combination corresponding to the shortest distance information among the fifteen pieces of distance information from the fifteen combinations and may repeat the above process using the remaining fourteen combinations.

Although a process of measuring a distance based on the second transceivers 510 included in the source assembly 110 has been described above, the present disclosure is not limited thereto. The detector controller may obtain a distance from one of the plurality of first transceivers to each of the plurality of second transceivers. The detector controller may obtain first distance information between the second transceiver and the first transceiver based on a signal between the second transceiver and the first transceiver. The first distance information may be a distance between the second transceiver and the first transceiver or may include an SID. However, the present disclosure is not limited thereto, and the detector controller may obtain the SID based on the first distance information.

The detector controller may obtain a distance from the first-first transceiver 921 included in the first transceivers to each of the second-first transceiver 911, the second-second transceiver 912, the second-third transceiver 913, and the second-fourth transceiver 914. In addition, the detector controller may match the plurality of first transceivers and the plurality of second transceivers one-to-one by matching one of the plurality of second transceivers to one of the plurality of first transceivers that is the closest thereto. For example, when transmitting a signal to the first-first transceiver 921, the second-first transceiver 911, the second-second transceiver 912, the second-third transceiver 913, and the second-fourth transceiver 914 may also send a transmission time. The main controller 300 may obtain the distance from the first-first transceiver 921 to each of the second-first transceiver 911, the second-second transceiver 912, the second-third transceiver 913, and the second-fourth transceiver 914 based on the time at which the signal is transmitted from the second-first transceiver 911, the second-second transceiver 912, the second-third transceiver 913, and the second-fourth transceiver 914 and the time at which the signal is received by the first-first transceiver 921. The detector controller may match the second-first transceiver 911, which is the closest to the first-first transceiver 921, to the first-first transceiver 921. Likewise, the detector controller may match the second-second transceiver 912, which is the closest to the first-second transceiver 922, to the first-second transceiver 922. The detector controller may match the second-third transceiver 913, which is the closest to the first-third transceiver 923, to the first-third transceiver 923. The detector controller may match the second-fourth transceiver 914, which is the closest to the first-fourth transceiver 924, to the first-fourth transceiver 924.

As above, at least one of the main controller 300 and the detector controller may obtain the SID from the source assembly to the detector based on a plurality of distances between the plurality of first transceivers and the plurality of second transceivers that correspond one-to-one. More specifically, at least one of the main controller 300 and the detector controller may obtain first distance information using one of a mean value, a minimum value, a maximum value, and a median value of the plurality of distances. The main controller 300 may obtain an SID 940 from a corrected first distance based on at least one of an SID generation table and an SID generation function.

The subject 210 of FIG. 9 may be smaller than the detector. Accordingly, the subject may not block a signal between the first transceiver 410 and the second transceiver 510. However, when the subject 210 is larger than the detector as in FIG. 10, the subject may block a signal between the first transceiver 410 and the second transceiver 510. Even when the subject 210 blocks the signal, there may be no problem in matching the plurality of first transceivers 410 and the plurality of second transceivers 510 one-to-one. However, when the subject 210 blocks the signal, there may be a problem in measuring the SID 940 by the main controller 300. This is because, since the signal between the first transceiver 410 and the second transceiver 510 is blocked by the subject 210, it is difficult for the main controller 300 to measure a linear distance between the first transceiver 410 and the second transceiver 510. In order to address this, the main controller 300 may measure first distance information between the second transceiver and the first transceiver. The first distance information may be a distance between the first transceiver and the second transceiver that correspond to each other. Here, the first distance information may be a distance with an error generated due to blocking by the subject.

The main controller 300 may guide to move the source assembly 110 so that the source assembly 110 and the detector 120 are not blocked from each other by the subject. The user may move the source assembly 110 for a moment based on the guide. The medical imaging device 100 may obtain the position of the detector from a third transceiver disposed at a fixed position in a ward. A separate beacon including the third transceiver may be located at the fixed position in the ward. However, the present disclosure is not limited thereto, and the third transceiver may be included in the main body 130 of the medical imaging device 100. This is because, even when the source assembly 110 moves, the main body 130 may be fixed inside the ward. The main controller 300 may guide the source assembly 110 to move back to an imaging position. The main controller 300 may determine the position of the source assembly 110 relative to the third transceiver at the imaging position. The main controller 300 may display the detector region 730 in the 3D camera image 710 based on the position of the detector 120 and the position of the source assembly 110 from the third transceiver. In addition, the main controller 300 may determine the SID based on the position of the detector 120 and the position of the source assembly 110 from the third transceiver. However, the present disclosure is not limited thereto, and the medical imaging device 100 may perform the following process.

If a differential between a plurality of pieces of first distance information measured using the plurality of first transceivers and the plurality of second transceivers is greater than or equal to a threshold distance, the main controller 300 may output a message that guides to move the source assembly 110. Here, the first distance information may include at least one of first-first distance information, first-second distance information, first-third distance information, and first-fourth distance information. When the differential between the plurality of measured pieces of first distance information is smaller than the threshold distance, the main controller 300 may measure an SOD 930, which is a distance from the source assembly 110 to the subject 210, based on the 3D camera 520. The SOD 930 may be included in second distance information, but the present disclosure is not limited thereto.

The main controller 300 may determine a distance to an object located the closest to the 3D camera 520 as the SOD 930. In addition, the main controller 300 may determine a distance from at least one of the radiation irradiation region 720 and the detector region 730 to the object located the closest to the 3D camera 520 as the SOD 930. The main controller 300 may correct the first distance based on the SOD 930. The main controller 300 may obtain the first distance corrected according to the SOD 930 based on at least one of a first distance changing table and a first distance changing function. For example, the first distance changing table and the first distance changing function may generate the corrected first distance by decreasing the first distance further with a shorter SOD 930. This is because a short SOD 930 indicates that the subject 210 is thick. In addition, the main controller 300 may determine the SID 940 based on the corrected first distance. The main controller 300 may obtain the SID 940 from the corrected first distance based on at least one of the SID generation table and the SID generation function.

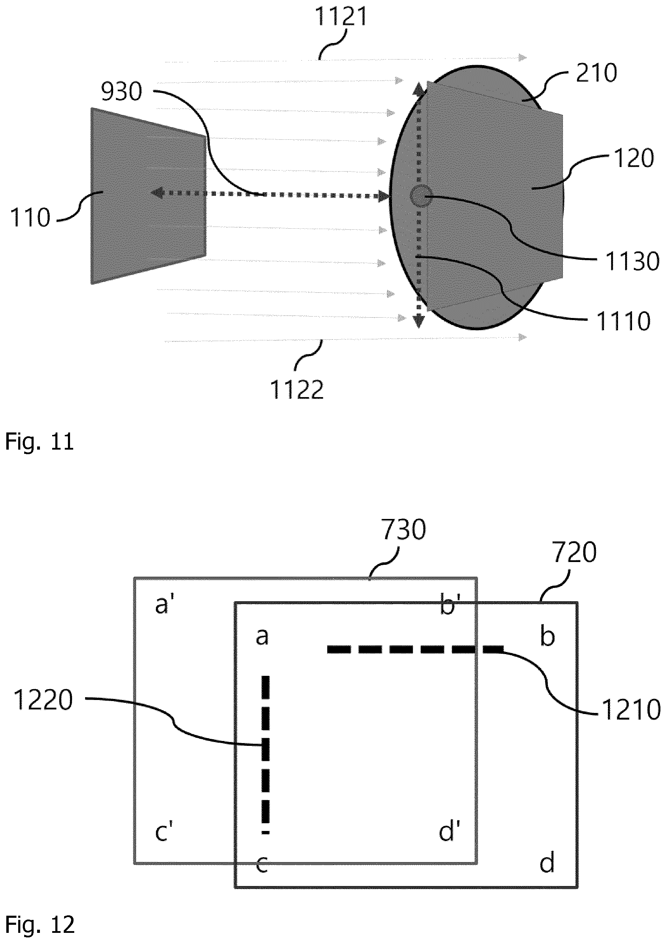

FIG. 11 is a view for describing an operation of the medical imaging device according to one embodiment of the present disclosure.

As described above, the main controller 300 may measure a distance between the front surface of the source assembly 110 and an object shown in the 3D camera image 710 using the 3D camera 520. The user may place the source assembly 110 to face the subject 210 in order to obtain a medical image. The main controller 300 may perform a step of obtaining second distance information from the 3D camera 520 to at least one object included in the 3D camera image. The main controller 300 may perform a step of obtaining second distance information from the 3D camera 520 to at least one object included in at least one of the radiation irradiation region 720 and the detector region 730 of the 3D camera image 710. The main controller 300 may obtain the SOD from the source assembly to the subject based on the second distance information. For example, the main controller 300 may determine the shortest distance among pieces of second distance information as the SOD 930. This is because the user will place the subject to be the closest to the source assembly 110. The SOD may be a distance from a source to a subject.

In addition, the medical imaging device 100 may obtain a width 1110 of the subject 210. For example, when distances 1121 and 1122 between the source assembly 110 and an object shown in the 3D camera image 710 are greater than a predetermined subject threshold distance, the main controller 300 may remove the object and may determine a subject region. In addition, the main controller 300 may determine a width of the subject region as the width 1110 of the subject 210. For example, in FIG. 11, the width 1110 may indicate the width of the subject. In addition, the main controller 300 may determine a center 1130 of the subject region as the center 731 of the detector region 730. Accordingly, the medical imaging device 100 may determine the center 731 of the detector region 730 even when the detector 120 is blocked from view by the subject 210.

According to various embodiments of the present disclosure, the main controller 300 may determine a thickness of the subject. For example, the main controller 300 may obtain first distance information between the second transceiver 510 and the first transceiver 410 based on a signal between the second transceiver 510 and the first transceiver 410. In addition, the main controller 300 may obtain the SID from the source assembly to the detector based on the first distance. The main controller 300 may determine the thickness of the subject based on the SOD and the SID. For example, the main controller 300 may determine the thickness of the subject by subtracting the SOD from the SID. The main controller 300 may determine radiation irradiation setting information based on the thickness of the subject. The main controller 300 may pre-store a radiation irradiation table in which different pieces of radiation irradiation setting information are matched with different thicknesses of a subject. The main controller 300 may obtain radiation irradiation setting information corresponding to the thickness of the subject from the radiation irradiation table. For example, the radiation intensity may be higher or the radiation irradiation time may be longer with a thicker thickness of the subject. Since the medical imaging device 100 of the present disclosure automatically determines the thickness of the subject and determines the radiation irradiation setting information corresponding thereto, a high-quality medical image can be obtained, and user convenience can be improved.

According to various embodiments of the present disclosure, the following embodiment may also be possible. The subject and the hospital bed 220 may be included in the 3D camera image. In addition, a distance from the source assembly 110 to the hospital bed 220 may be similar to the distance from the source assembly 110 to the detector 120.

Accordingly, the main controller 300 may obtain both the SOD and the SID based on the second distance information, which is a distance between at least one object shown in the 3D camera image 710 and the source assembly 110. The main controller 300 may determine the SOD and the SID based on the second distance information based on a user input or a machine learning model. The machine learning model may be a model for determining whether only the subject and the hospital bed 220 are present in the 3D camera image. The main controller 300 may determine the smallest value among pieces of second distance information as the SOD. In addition, the main controller 300 may determine the greatest value among the pieces of second distance information as the SID.

A process for obtaining first alignment information, second alignment information, and third alignment information will be described below with reference to FIGS. 12 and 13. Operations of the medical imaging device according to FIGS. 12 and 13 may be performed prior to step (650) or together with step (650).

As described above, when performing step (650), the main controller 300 may determine alignment information related to at least one of the direction, distance, and angle of movement of the source assembly that is necessary to align the detector and the source assembly, based on at least one of the 3D camera, the first transceiver 410, and the second transceiver 510. Here, aligning the detector 120 and the source assembly 110 may mean letting the irradiation region and the detector region coincide or letting a line connecting the center of the detector and the center of the source assembly 110 be parallel to a radiation irradiation direction. The alignment information may include first alignment information, second alignment information, and third alignment information. The plurality of first transceivers may include the first-first transceiver 921, the first-second transceiver 922, the first-third transceiver 923, and the first-fourth transceiver 924. In addition, the plurality of second transceivers may include the second-first transceiver 911, the second-second transceiver 912, the second-third transceiver 913, and the second-fourth transceiver 914. The main controller 300 may measure a distance of one of the plurality of second transceivers relative to at least three of the plurality of first transceivers. In addition, the main controller 300 may measure a distance of one of the plurality of first transceivers relative to at least three of the plurality of second transceivers. The distance may be measured based on the time taken for a signal to move between one of the plurality of first transceivers and one of the plurality of second transceivers. The main controller 300 may determine the positions (coordinates) of the first transceivers 410 included in the detector 120 relative to the second transceivers 510 included in the source assembly 110 using triangulation. In addition, the main controller 300 may determine the positions (coordinates) of the second transceivers 510 included in the source assembly 110 relative to the first transceivers 410 included in the detector 120 using triangulation. Alternatively, the main controller 300 may obtain the position (coordinates) of the source assembly 110 relative to the detector 120. The main controller 300 may determine alignment information according to which the source assembly has to move for the detector 120 and the source assembly 110 to be aligned, based on the position of the source assembly 110 relative to the detector 120. That is, the main controller 300 may obtain at least one of the first alignment information, the second alignment information, and the third alignment information based on the first transceivers 410 and the second transceivers 510. A process of obtaining at least one of the first alignment information, the second alignment information, and the third alignment information will be described below with reference to FIGS. 12 and 13.

Although the process of obtaining alignment information using the first transceivers 410 and the second transceivers 510 has been described above, the present disclosure is not limited thereto. When determining alignment information, the main controller 300 may obtain a set of coordinates of the plurality of first transceivers and a set of coordinates of the plurality of second transceivers from the 3D camera image based on the 3D camera 520. For example, since the 3D camera 520 is included in the source assembly 110, the main controller 300 may determine predetermined positions in the 3D camera image as the set of the coordinates of the plurality of second transceivers. In addition, the main controller 300 may, after finding the detector region, determine predetermined positions in the detector region as the set of the coordinates of the plurality of first transceivers. In addition, the main controller 300 may obtain at least one of the first alignment information, the second alignment information, and the third alignment information using a method described below with reference to FIGS. 12 and 13.

FIG. 12 is a view for describing an operation of the medical imaging device according to one embodiment of the present disclosure.

When determining alignment information, the main controller 300 may measure an angle formed between the front surface of the source assembly and the surface of the detector based on at least one of the first transceiver 410, the second transceiver 510, the 3D camera 520, and the gyro sensor. Based on the angle formed between the front surface of the source assembly and the surface of the detector, the main controller 300 may output an angle that the source assembly should rotate (an angle of rotation of the source assembly) for a predetermined angle to be formed between the front surface of the source assembly and the surface of the detector. The predetermined angle may be an angle that should be formed between the front surface of the source assembly and the surface of the detector in order to capture a medical image. The predetermined angle may include an angle that allows the front surface of the source assembly and the surface of the detector to be parallel to each other, and the predetermined angle in this case may be 0°, for example. The predetermined angle may be greater than or equal to 0° and smaller than 90°. In addition, the predetermined angle may be determined based on a user input. This process will be described in more detail below.

According to one embodiment of the present disclosure, the main controller 300 may measure the angle formed between the front surface of the source assembly and the surface of the detector based on the plurality of first transceivers and the plurality of second transceivers. At least one of the posture information of the detector and the posture information of the source assembly may include the angle formed between the front surface of the source assembly and the surface of the detector. A process of obtaining the angle formed between the front surface of the source assembly and the surface of the detector will be described below.

The detector 120 may include the plurality of first transceivers 410. The plurality of first transceivers may include the first-first transceiver 921, the first-second transceiver 922, the first-third transceiver 923, and the first-fourth transceiver 924. The set of the coordinates of the plurality of first transceivers may be expressed as below.

Set of coordinates of plurality of first transceivers= {(x(a'), y(a')), (x(b'), y(b')), (x(c'), y(c')), (x(d'), y(d'))}

The coordinates may be coordinates at each point on the display of the medical imaging device or may be coordinates relative to a predetermined origin in an actual space. The predetermined origin may be located on the source assembly or the detector.

When the origin of the coordinate system is on the source assembly 110, the main controller 300 may determine the set of the coordinates of the plurality of first transceivers by triangulation using the plurality of first transceivers and the plurality of second transceivers. When the origin of the coordinate system is on the detector 120, the main controller 300 may obtain a predetermined set of coordinates of the plurality of first transceivers from a memory.

Likewise, the source assembly 110 may include the plurality of second transceivers 510. The plurality of second transceivers may include the second-first transceiver 911, the second-second transceiver 912, the second-third transceiver 913, and the second-fourth transceiver 914. The set of the coordinates of the plurality of second transceivers may be expressed as below.

Set of coordinates of plurality of second transceivers= {(x(a), y(a)), (x(b), y(b)), (x(c), y(c)), (x(d), y(d))}

The coordinates of the plurality of first transceivers and the coordinates of the plurality of second transceivers may be expressed with values in the same coordinate system. When the origin of the coordinate system is on the source assembly 110, the main controller 300 may obtain a predetermined set of coordinates of the plurality of second transceivers from a memory. When the origin of the coordinate system is on the detector 120, the main controller 300 may determine the set of the coordinates of the plurality of second transceivers by triangulation using the plurality of first transceivers and the plurality of second transceivers.

As described above, when the plurality of first transceivers 410 and the plurality of second transceivers 510 correspond one-to-one, the medical imaging device 100 may perform a step of obtaining first alignment information of the source assembly 110 for adjusting the radiation irradiation region 720 of the source assembly 110 to include the detector region 730 based on the posture information of the detector 120. For example, the plurality of first transceivers 410 may include the first-first transceiver 921 and the first-second transceiver 922. In addition, the plurality of second transceivers 510 may include the second-first transceiver 911 corresponding to the first-first transceiver 921 and the second-second transceiver 912 corresponding to the first-second transceiver 922. The main controller 300 may obtain a first-first distance between the first-first transceiver 921 and the second-first transceiver 911. The main controller 300 may obtain a first-second distance between the first-second transceiver 922 and the second-second transceiver 912. When a difference between the first-first distance and the first-second distance is greater than or equal to a predetermined threshold distance, the main controller 300 may output a message that guides to move the source assembly 110 and adjust the first-first distance and the first-second distance to be similar to each other. Here, the predetermined threshold distance may be a value stored in the memory. The predetermined threshold distance may be a value for determining whether the first-first distance and the first-second distance significantly differ from each other. The first-first distance and the first-second distance significantly differing from each other may mean that the detector 120 and the source assembly 110 are not aligned.

The message that guides to move the source assembly 110 and adjust the first-first distance and the first-second distance to be similar to each other may be output based on obtaining of third alignment information. The third alignment information may be a direction of rotating the source assembly 110 about an axis perpendicular to the longitudinal direction of the source arm 140. Referring to FIG. 12, the main controller 300 may obtain a horizontal differential depth and a vertical differential depth as below.

$$\text{Horizontal differential depth} = \text{depth}(a\text{-}a') - \text{depth}(b\text{-}b')$$

$$\text{Vertical differential depth} = \text{depth}(a\text{-}a') - \text{depth}(c\text{-}c')$$

The horizontal differential depth and the vertical differential depth may correspond to the measured angle formed between the front surface of the source assembly and the surface of the detector. Here, when the value of the horizontal differential depth is 0, line a-b and line a'-b' may be parallel to each other.

Based on the angle formed between the front surface of the source assembly and the surface of the detector, the main controller 300 may obtain an angle of rotation of the source assembly for a predetermined angle to be formed between the front surface of the source assembly and the surface of the detector. Alignment information may include the angle of rotation of the source assembly. In addition, the main controller 300 may output the angle of rotation of the source assembly.

If the value of the horizontal differential depth is a positive number, the main controller 300 may obtain third alignment information for rotating the source assembly 110 so that distance b-b' increases or distance a-a' decreases based on a vertical axis 1220. If the value of the horizontal differential depth is a negative number, the main controller 300 may obtain third alignment information for rotating the source assembly 110 so that distance b-b' decreases or distance a-a' increases based on the vertical axis 1220. Although the vertical axis 1220 is arbitrarily drawn in FIG. 12, the present disclosure is not limited thereto, and the vertical axis 1220 may be located at a position different from that shown in FIG. 12.

In addition, the main controller 300 may obtain third alignment information also based on a predetermined angle. As described above, the predetermined angle may be a constant or may be obtained based on a user input. The main controller 300 may determine a depth offset based on the predetermined angle. The larger the predetermined angle, the larger the depth offset may be. When a value resulting from subtracting a depth offset value from the horizontal differential depth is 0, the predetermined angle may be formed between line a-b and line a'-b'. If the value resulting from subtracting the depth offset value from the horizontal differential depth is a positive number, the main controller 300 may obtain third alignment information for rotating the source assembly 110 so that distance b-b' increases or distance a-a' decreases based on a vertical axis 1220. If the value resulting from subtracting the depth offset value from the horizontal differential depth is a negative number, the main controller 300 may obtain third alignment information for rotating the source assembly 110 so that distance b-b' decreases or distance a-a' increases based on the vertical axis 1220. Although the vertical axis 1220 is arbitrarily drawn in FIG. 12, the present disclosure is not limited thereto, and the vertical axis 1220 may be located at a position different from that shown in FIG. 12.

In addition, when the value of the vertical differential depth is 0, line a-c and line a'-c' may be parallel to each other. If the value of the vertical differential depth is a positive number, the main controller 300 may obtain third alignment information for rotating the source assembly 110 so that distance c-c' increases or distance a-a' decreases based on a horizontal axis 1210. If the value of the vertical differential depth is a negative number, the main controller 300 may obtain third alignment information for rotating the source assembly 110 so that distance c-c' decreases or distance a-a' increases based on the horizontal axis 1210.

In addition, the main controller 300 may obtain third alignment information also based on a predetermined angle. For example, when a value resulting from subtracting a depth offset value from the vertical differential depth is 0, the predetermined angle may be formed between line a-c and line a'-c'. If the value resulting from subtracting the depth offset value from the vertical differential depth is a positive number, the main controller 300 may obtain third alignment information for rotating the source assembly 110 so that distance c-c' increases or distance a-a' decreases based on the horizontal axis 1210. If the value resulting from subtracting the depth offset value from the vertical differential depth is a negative number, the main controller 300 may obtain third alignment information for rotating the source assembly 110 so that distance c-c' decreases or distance a-a' increases based on the horizontal axis 1210.

In the above, the third alignment information is determined using the first transceivers 410 and the second transceivers 510. However, the present disclosure is not limited thereto. When determining the alignment information, the main controller 300 may measure the angle formed between the front surface of the source assembly and the surface of the detector based on at least one of the 3D camera 520 and the gyro sensor 530. As described above, the 3D camera 520 may determine the detector region 730 using a machine learning model. In addition, the main controller 300 may also determine a slope of the detector region using depth information measured by the 3D camera 520. In addition, the main controller 300 may also obtain depth(a-a'), depth(b-b'), and depth(c-c') using the depth information measured by the 3D camera 520. The main controller 300 may obtain the horizontal differential depth and the vertical differential depth based on the 3D camera 520, and may obtain the third alignment information.

The slope of the detector region may also be measured by the gyro sensor included in the detector. In the present disclosure, the slope of the detector 120 may be a slope based on a fixed surface. The fixed surface may be the ground, for example. Alternatively, the slope of the detector 120 may be a slope based on the radiation irradiation surface of the source assembly.

The gyro sensor 530 of the source assembly 110 may measure the slope of the source assembly. In the present disclosure, the slope of the source assembly 110 may be a slope based on a fixed surface. The fixed surface may be the ground, for example. Alternatively, the slope of the source assembly 110 may be a slope based on the surface of the detector.

When outputting a message that guides to adjust the irradiation region and the detector region, the main controller 300 may output an angle that the source assembly should rotate (an angle of rotation of the source assembly) for the front surface of the source assembly and the surface of the detector to be parallel based on the angle formed between the front surface of the source assembly and the surface of the detector. The angle that the source assembly should rotate (the angle of rotation of the source assembly) may be included in the third alignment information. For example, the main controller 300 may obtain the slope of the source assembly 110 relative to the detector and may allow the source assembly 110 to rotate in the opposite direction of the slope to guide the front surface of the source assembly and the surface of the detector to be parallel to each other.

FIG. 13 is a view for describing an operation of the medical imaging device according to one embodiment of the present disclosure.

When determining alignment information, the main controller 300 may obtain position information of a predetermined first alignment point of the irradiation region and a predetermined second alignment point of the detector region in the 3D camera image based on at least one of the first transceiver, the second transceiver, the 3D camera, and the gyro sensor. The position information may include at least one of coordinate values of the predetermined first alignment point of the irradiation region and coordinate values of the predetermined second alignment point of the detector region. The position information may be information expressed in the coordinate system of the 3D camera image, but is not limited thereto. The position information may be information expressed in a coordinate system of a 3D space. For example, the position information may be expressed in the coordinate system with one point of at least one of the source assembly 110 and the main body 130 as the origin. When the coordinate systems of the coordinate values are different from each other, the main controller 300 may perform coordinate transformation of the coordinate values and may allow the coordinate values to match one of the coordinate system of the 3D camera image and the coordinate system of the 3D space.

The main controller 300 may output at least one of the direction and distance of movement of the source assembly that is necessary for the irradiation region and the detector region to match based on the position information. An operation of the main controller 300 will be described in more detail below.

A first alignment point may be the same as at least one of the points a, b, c, and d. In addition, the first alignment point may correspond to the position of at least one second transceiver 510. A second alignment point may be the same as at least one of the points a', b', c', and d'. The second alignment point may correspond to the position of at least one first transceiver 410.

When outputting the message that guides to adjust the irradiation region and the detector region, the main controller 300 may output at least one of the direction and distance of movement of the source assembly that is necessary for the irradiation region and the detector region to match based on the position information. At least one of the direction and distance of movement of the source assembly may be included in at least one of the first alignment information and the second alignment information.

FIG. 13 will be described in more detail below. The main controller 300 may obtain the position information based on the plurality of first transceivers and the plurality of second transceivers. At least one of the posture information of the detector and the posture information of the source assembly may include the position information.

The detector 120 may include the plurality of first transceivers 410. The plurality of first transceivers may include the first-first transceiver 921, the first-second transceiver 922, the first-third transceiver 923, and the first-fourth transceiver 924. The set of the coordinates of the plurality of first transceivers may be expressed as below. Since the method of obtaining the set of the coordinates of the plurality of first transceivers has been described above, repeated description thereof will be omitted.

Set of coordinates of plurality of first transceivers= {(x(a'), y(a')), (x(b'), y(b')), (x(c'), y(c')), (x(d'), y(d'))}

Likewise, the source assembly 110 may include the plurality of second transceivers 510. The plurality of second transceivers may include the second-first transceiver 911, the second-second transceiver 912, the second-third transceiver 913, and the second-fourth transceiver 914. The set of the coordinates of the plurality of second transceivers may be expressed as below. Since the method of obtaining the set of the coordinates of the plurality of first transceivers has been described above, repeated description thereof will be omitted.

Set of coordinates of plurality of second transceivers= {(x(a), y(a)), (x(b), y(b)), (x(c), y(c)), (x(d), y(d))}

The main controller 300 may determine Alignment x and Alignment y, which are pieces of position information, as below.

$$\text{Alignment x} = ([x(a)\text{-}x(a')] + [x(b)\text{-}x(b')] + [x(c)\text{-}x(c')] + [x(d)\text{-}x(d')])/n \text{ (here, } n = 4)$$

$$\text{Alignment y} = ([y(a)\text{-}y(a')] + [y(b)\text{-}y(b')] + [y(c)\text{-}y(c')] + [y(d)\text{-}y(d')])/n \text{ (here, } n = 4)$$

In addition, the main controller 300 may obtain at least one of the direction and distance of movement of the source assembly that is necessary for the irradiation region and the detector region to match based on the position information. Alignment information may include at least one of the direction and distance of movement of the source assembly The main controller 300 may output at least one of the direction and distance of movement of the source assembly.

If Alignment x is a negative number, a direction of the first alignment information may indicate rightward movement as much as |Alignment x|

In addition, if Alignment x is a positive number, the first alignment information may indicate leftward movement as much as |Alignment x|

In addition, if Alignment y is a negative number, the first alignment information may indicate upward movement as much as |Alignment y|

If Alignment y is a positive number, the first alignment information may indicate downward movement as much as | Alignment y|

In addition, the absolute value of Alignment x may be related to a distance that the source assembly 110 should move in order to align the source assembly 110 and the detector. The main controller 300 may pre-store a moving distance obtaining table and a moving distance obtaining function in which different absolute values of Alignment x and different distances that the source assembly 110 should move are matched. The main controller 300 may obtain a distance that the source assembly 110 should move that corresponds to the absolute value of Alignment x, based on the moving distance obtaining table and the moving distance obtaining function. The main controller 300 may include the direction and distance of necessary movement in the first alignment information. In addition, the main controller 300 may display the first alignment information on the display. The user may move the source assembly 110 based on the first alignment information, and accordingly, the irradiation region and the detector region may coincide, or the center of the detector and the center of the irradiation region may coincide.

The plurality of first transceivers 410 and the plurality of second transceivers 510 may be implemented using UWB. UWB may be used for the purpose of obtaining x and y coordinates. Since medical image quality may degrade when the detector 120 and the source assembly 110 are not aligned, the medical imaging device 100 may take approximate values of mean values as above to guide alignment between the detector 120 and the source assembly 110.

Although the process of obtaining the first alignment information using the first transceivers 410 and the second transceivers 510 has been described above, the present disclosure is not limited thereto. When determining the alignment information, the main controller 300 may obtain a set of coordinates of the plurality of first transceivers and a set of coordinates of the plurality of second transceivers from the 3D camera image based on the 3D camera 520. For example, since the 3D camera 520 is included in the source assembly 110, the main controller 300 may determine predetermined positions in the 3D camera image as the set of the coordinates of the plurality of second transceivers. In addition, the main controller 300 may, after finding the detector region, determine predetermined positions in the detector region as the set of the coordinates of the plurality of first transceivers. In addition, the main controller 300 may obtain the first alignment information according to the method described above.

The main controller 300 may obtain body part identification information of a subject. The body part identification information of the subject may be obtained based on a user input or pre-stored information on the subject. The main controller 300 may model the subject three-dimensionally or two-dimensionally based on the body part identification information and the SOD to obtain a body part model. The SOD may be determined using the method described above. In addition, the main controller 300 may place the center of the detector region at the center of the body part model shown in the 3D camera image. The center of the body part model may include at least one of the position of the center of gravity of the body part model or the center of a rectangle circumscribing the body part model.

However, the present disclosure is not limited thereto, and as described above with reference to FIG. 11, the center 731 of the detector region 730 may be placed at the center 1130 of the subject region. The main controller 300 may determine the detector region 730 based on the SID. The SID may be obtained using the method described above. The size of the detector region 730 may be determined in the 3D camera image based on the SID. When the SID is large, since it indicates that the distance between the source assembly 110 and the detector 120 is large, the detector region 730 may be small in the 3D camera image. In addition, when the SID is small, since it indicates that the distance between the source assembly 110 and the detector 120 is small, the detector region 730 may be large in the 3D camera image. The main controller 300 may determine the size of the detector according to at least one of the first distance information and the SID based on at least one of the detector size table and the detector size function.

To sum up the above description, the medical imaging device 100 may obtain at least one of the first alignment information, the second alignment information, and the third alignment information of the source assembly 110. In addition, the medical imaging device 100 may display at least one of the first alignment information, the second alignment information, and the third alignment information of the source assembly 110 on the display to allow the user to align the source assembly 110 to the detector 120. In addition, the medical imaging device 100 may automatically align the source assembly 110 and the detector 120 based on at least one of the first alignment information, the second alignment information, and the third alignment information. More specifically, the source arm 140 of the medical imaging device 100 may be a robot arm, and the source arm 140 may move the source assembly 110 based on at least one of the first alignment information, the second alignment information, and the third alignment information to automatically align the source assembly 110 and the detector 120.

The direction of the first alignment information may be a direction in which the source assembly 110 is moved on a plane parallel to the front surface of the source assembly 110. Referring to FIG. 7, the direction of the first alignment information may be displayed as the arrow 741 and the arrow 742 on the display. The first alignment information may be a direction for moving the source assembly 110 so that the radiation irradiation region 720 includes the detector region 730. In the present disclosure, the first alignment information may be information including not only the direction but also the distance. The distance of the first alignment information may be displayed around the arrow 741 and the arrow 742. Accordingly, the user may align the source assembly 110 and the detector 120 while looking at the display on which the first alignment information is displayed.

The second alignment information may be a direction for adjusting the distance between the source assembly 110 and the detector 120. That is, the second alignment information may be parallel to a direction from the center of the source assembly 110 toward the center of the detector 120. The second alignment information may affect at least one of the SID and the SOD. In the present disclosure, the second alignment information may be information including not only the direction but also the distance. At least one of the direction and distance of the second alignment information may be displayed on the display. Accordingly, the user may adjust the distance between the source assembly 110 and the detector 120 while looking at the display.

The third alignment information may be a direction of rotating the source assembly 110 about the horizontal axis 1210 and the vertical axis 1220 that are parallel to the plane perpendicular to the longitudinal direction of the source arm 140. For example, the horizontal axis 1210 may correspond to the pitch axis, and the vertical axis 1220 may correspond to the yaw axis. However, the present disclosure is not limited thereto, and the horizontal axis 1210 may correspond to the yaw axis, and the vertical axis 1220 may correspond to the pitch axis. The third alignment information may be a direction of rotating the source assembly 110 to make the radiation irradiation direction of the source assembly 110 perpendicular to the surface of the detector 120. In other words, the third alignment information may be a direction of rotating the source assembly 110 to make the front surface of the source assembly 110 parallel to the surface of the detector 120. In the present disclosure, the third alignment information may be information including not only the direction but also an angle of rotation. In addition, although the third alignment information has been described above based on the horizontal axis 1210 and the vertical axis 1220, the present disclosure is not limited thereto, and the third alignment information may be information further including a direction of rotation and an angle of rotation about the roll axis. The main controller 300 may display the third alignment information on the display. The user may adjust the radiation irradiation direction of the source assembly 110 and the surface of the detector 120 to be perpendicular to each other while looking at the display.

The medical imaging device 100 may fix the moving direction of the source assembly 110 to use only one of the first alignment information, the second alignment information, and the third alignment information based on a selection by the user. For example, the medical imaging device 100 may display, on the display, an object for allowing the source assembly 110 to move according to one of the first alignment information, the second alignment information, and the third alignment information. The object may be an image that includes at least one of text, color, and shape and is displayed on the display. When the user has selected an object related to the first alignment information, the source assembly 110 can move only in the direction parallel to the direction of the first alignment information and may be prevented from moving in the directions of the second alignment information and the third alignment information. For example, the main controller 300 may fix joints for moving in the directions of the second alignment information and the third alignment information. Likewise, when the user has selected an object related to the second alignment information, the source assembly 110 can move only in the direction parallel to the direction of the second alignment information and may be prevented from moving in the directions of the first alignment information and the third alignment information. In addition, when the user has selected an object related to the third alignment information, the source assembly 110 can move only in the direction parallel to the direction of the third alignment information and may be prevented from moving in the directions of the second alignment information and the first alignment information.

The medical imaging device according to one embodiment of the present disclosure can have high alignment accuracy because the detector and the source assembly are aligned using at least one sensor. In addition, during alignment, since the medical imaging device 100 outputs at least one of the first alignment information, the second alignment information, and the third alignment information for alignment, and user intervention is minimized, user convenience can be improved. In addition, since the source assembly 110 can automatically move to an alignment position by the robot arm based on the first alignment information, the second alignment information, or the third alignment information, user convenience can be enhanced, and a medical image can be rapidly obtained.

According to the medical imaging device 100 described above, the source assembly 110 and the detector 120 can be rapidly and precisely aligned. In addition, a burden due to imaging can be reduced for both the user and the patient. In addition, an inconvenience of having to perform imaging again can be reduced, and exposure to radiation due to performing imaging again can be eliminated.

The present disclosure has been described above using various embodiments. Those of ordinary skill in the art to which the present invention pertains should understand that the present invention can be implemented in modified forms within the scope not departing from essential characteristics of the present invention. Therefore, the embodiments disclosed herein should be considered illustrative instead of limiting. The scope of the present invention is shown by the claims rather than the above description, and all differences within the scope equivalent to the claims should be construed as belonging to the present invention.

Meanwhile, the above-described embodiments of the present invention can be written by a computer executable program and may be implemented in a universal digital computer running the program using computer readable recording media. The computer readable recording media include storage media such as magnetic storage media (e.g., a read-only memory (ROM), a floppy disk, a hard disk, etc.) and optical readable media (e.g., a CD-ROM, a DVD, etc.).

## Claims

1.  A medical imaging device comprising:

    a detector including a detector controller for controlling an operation of the detector and a first transceiver disposed at a predetermined position;
    a source assembly including a second transceiver disposed on a front surface; and
    a main controller controlling an operation of the medical imaging device,
    wherein the main controller obtains alignment information for alignment between the detector and the source assembly.

2.  The medical imaging device of claim 1, wherein the alignment information includes information related to at least one of a direction, a distance, and an angle of movement of the source assembly that is necessary, based on at least one of posture information of the detector and posture information of the source assembly.

3.  The medical imaging device of claim 1, wherein, when a region of the detector and an irradiation region coincide based on the alignment information, the medical imaging device outputs a signal indicating that the region of the detector and the irradiation region coincide.

4.  The medical imaging device of claim 1, wherein:

    the detector includes a plurality of first transceivers;
    the source assembly includes a plurality of second transceivers; and
    the main controller obtains a source-to-image distance (SID) from the source assembly to the detector based on a plurality of distances between the plurality of first transceivers and the plurality of second transceivers that correspond one-to-one.

5.  The medical imaging device of claim 1, wherein:

    the source assembly further includes a 3D camera for obtaining a 3D camera image; and
    the main controller obtains at least one of the posture information of the source assembly and the posture information of the detector based on the 3D camera image.

6.  The medical imaging device of claim 5, wherein the main controller:

obtains pre-alignment information using the first transceiver and the second transceiver;

obtains post-alignment information based on the 3D camera image; and

determines final alignment information based on the pre-alignment information and the post-alignment information.

7. The medical imaging device of claim 5, wherein the medical imaging device:

obtains first distance information between the first transceiver and the second transceiver based on a signal between the first transceiver and the second transceiver; and

displays the region of the detector on the 3D camera image based on the first distance information.

8. The medical imaging device of claim 5, wherein the main controller:

displays the region of the detector in the 3D camera image;

obtains second distance information from the 3D camera to at least one image-captured object; and

obtains a source-to-image distance (SID) from the source assembly to the detector based on the region of the detector and the second distance information.

9. The medical imaging device of claim 5, wherein the main controller:

obtains second distance information from the 3D camera to at least one image-captured object; and

obtains a source-to-object distance (SOD) from the source assembly to a subject based on the second distance information.

10. The medical imaging device of claim 9, wherein the main controller:

obtains first distance information between the first transceiver and the second transceiver based on a signal between the first transceiver and the second transceiver;

obtains a source-to-image distance (SID) from the source assembly to the detector based on the first distance information; and

determines a thickness of the subject based on the SOD and the SID.

11. The medical imaging device of claim 10, wherein the main controller:

obtains body part identification information of the subject;

obtains a body part model by modeling the subject three-dimensionally or two-dimensionally based on the body part identification information and the SOD;

places the center of the region of the detector at the center of the body part model shown in the 3D camera image; and

displays the region of the detector based on the SID.

12. The medical imaging device of claim 5, wherein the 3D camera includes at least one of a depth measurement camera, a red-green-blue-depth (RGBD) camera, or a time-of-flight (TOF) camera.

Fig. 1

Fig. 2

Fig. 3

120

410

410

410

410

Fig. 4

Fig. 5

START

ACTIVATE MEDICAL IMAGING DEVICE — 610

OBTAIN 3D CAMERA IMAGE — 620

DISPLAY IRRADIATION REGION — 630

DISPLAY DETECTOR REGION — 640

OUTPUT MESSAGE THAT GUIDES
TO ADJUST DETECTOR REGION — 650

TRANSMIT RADIATION IRRADIATION
READY SIGNAL TO FIRST TRANSCEIVER — 660

END

Fig. 6

Fig. 7

START

OBTAIN FIRST DISTANCE INFORMATION — 810

DETERMINE SIZE OF DETECTOR — 820

DISPLAY DETECTOR REGION
IN 3D CAMERA IMAGE — 830

END

Fig. 8

Fig. 9

Fig. 10

Fig. 11

Fig. 12

730

720

a'

b'

a

b

c'

d'

c

d

Fig. 13

1410 1420 1430 1440 1450 1460 1470

Fig. 14

## INTERNATIONAL SEARCH REPORT

| International application No. |
|---|
| **PCT/KR2024/007155** |

| A. | CLASSIFICATION OF SUBJECT MATTER |
|---|---|

**A61B 6/58**(2024.01)i; **A61B 6/00**(2006.01)i

According to International Patent Classification (IPC) or to both national classification and IPC

| B. | FIELDS SEARCHED |
|---|---|

Minimum documentation searched (classification system followed by classification symbols)

A61B 6/58(2024.01); A61B 6/00(2006.01); A61B 6/03(2006.01); A61B 6/14(2006.01); G01N 23/04(2006.01); G01T 1/161(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 엑스선(x-ray), 디텍터(detector), 통신(communication), 정렬(alignment), 제어(control), 표시(display)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2016-0062279 A (SAMSUNG ELECTRONICS CO., LTD.) 02 June 2016 (2016-06-02)<br>See paragraphs [0123]-[0213]; claim 9; and figure 18. | 1-12 |
| Y | KR 10-2015-0040768 A (SAMSUNG ELECTRONICS CO., LTD.) 15 April 2015 (2015-04-15)<br>See paragraphs [0084] and [0153]; and claims 20 and 28. | 1-12 |
| Y | KR 10-2501712 B1 (SAMSUNG ELECTRONICS CO., LTD.) 21 February 2023 (2023-02-21)<br>See paragraph [0230]; and claim 17. | 9-11 |
| A | US 2014-0119500 A1 (FUJIFILM CORPORATION) 01 May 2014 (2014-05-01)<br>See claim 10. | 1-12 |
| A | US 2012-0307965 A1 (BOTHOREL et al.) 06 December 2012 (2012-12-06)<br>See claims 1, 5 and 9. | 1-12 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 September 2024** | **12 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2">INTERNATIONAL SEARCH REPORT<br>Information on patent family members</td><td>International application No.<br><br>**PCT/KR2024/007155**</td></tr>
</table>

| Patent document cited in search report | | | Publication date (day/month/year) | Patent family member(s) | | | Publication date (day/month/year) |
|---|---|---|---|---|---|---|---|
| KR | 10-2016-0062279 | A | 02 June 2016 | EP | 3223705 | A1 | 04 October 2017 |
| | | | | EP | 3223705 | A4 | 27 December 2017 |
| | | | | KR | 10-2328118 | B1 | 18 November 2021 |
| | | | | US | 10507001 | B2 | 17 December 2019 |
| | | | | US | 2017-0258434 | A1 | 14 September 2017 |
| | | | | WO | 2016-085211 | A1 | 02 June 2016 |
| KR | 10-2015-0040768 | A | 15 April 2015 | CN | 105592797 | A | 18 May 2016 |
| | | | | CN | 105592797 | B | 29 January 2019 |
| | | | | EP | 2856942 | A1 | 08 April 2015 |
| | | | | EP | 2856942 | B1 | 03 January 2018 |
| | | | | EP | 3351175 | A1 | 25 July 2018 |
| | | | | KR | 10-2017-0115018 | A | 16 October 2017 |
| | | | | US | 2015-0098551 | A1 | 09 April 2015 |
| | | | | US | 2015-0342558 | A1 | 03 December 2015 |
| | | | | US | 9134436 | B2 | 15 September 2015 |
| | | | | WO | 2015-053544 | A1 | 16 April 2015 |
| KR | 10-2501712 | B1 | 21 February 2023 | KR | 10-2016-0072024 | A | 22 June 2016 |
| US | 2014-0119500 | A1 | 01 May 2014 | CN | 103635139 | A | 12 March 2014 |
| | | | | EP | 2730226 | A1 | 14 May 2014 |
| | | | | EP | 2730226 | A4 | 08 April 2015 |
| | | | | US | 9380985 | B2 | 05 July 2016 |
| | | | | WO | 2013-005833 | A1 | 10 January 2013 |
| US | 2012-0307965 | A1 | 06 December 2012 | EP | 2713886 | A2 | 09 April 2014 |
| | | | | EP | 2713886 | A4 | 29 October 2014 |
| | | | | EP | 2713886 | B1 | 20 September 2017 |
| | | | | US | 8670521 | B2 | 11 March 2014 |
| | | | | WO | 2012-166262 | A2 | 06 December 2012 |
| | | | | WO | 2012-166262 | A3 | 24 January 2013 |

Form PCT/ISA/210 (patent family annex) (July 2022)